(19) Europäisches Patentamt
European Patent Office
Office européen des brevets

(11) EP 4 167 938 B1

(12) EUROPEAN PATENT SPECIFICATION

(45) Date of publication and mention of the grant of the patent:
**27.08.2025 Bulletin 2025/35**

(21) Application number: **21733945.6**

(22) Date of filing: **15.06.2021**

(51) International Patent Classification (IPC):
***A61K 8/35*** *(2006.01)* ***A61K 8/365*** *(2006.01)*
***A61K 8/41*** *(2006.01)* ***A61K 8/43*** *(2006.01)*
***A61K 8/44*** *(2006.01)* ***A61K 8/49*** *(2006.01)*
***A61Q 17/04*** *(2006.01)* ***C11D 7/26*** *(2006.01)*
***C11D 7/32*** *(2006.01)*

(52) Cooperative Patent Classification (CPC):
**A61K 8/35; A61K 8/365; A61K 8/416; A61K 8/43; A61K 8/44; A61K 8/4926; A61Q 17/04; C11D 3/20; C11D 3/42;** A61K 2800/522

(86) International application number:
**PCT/EP2021/066048**

(87) International publication number:
**WO 2021/254998 (23.12.2021 Gazette 2021/51)**

# (54) BIODEGRADABLE UV ABSORBERS

BIOLOGISCH ABBAUBARE UV-ABSORBER

ABSORBEURS D'UV BIODÉGRADABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2020 EP 20180528**

(43) Date of publication of application:
**26.04.2023 Bulletin 2023/17**

(73) Proprietor: **BASF SE**
**67056 Ludwigshafen am Rhein (DE)**

(72) Inventors:
- **GRUMELARD, Julie**
**79639 Grenzach-Wyhlen (DE)**
- **EHLIS, Thomas**
**4133 Schweizerhalle (Muttenz) (CH)**
- **GIESINGER, Jochen**
**79639 Grenzach-Wyhlen (DE)**
- **BROCK-NANNESTAD, Theis**
**2100 Copenhagen (DK)**
- **PEDERSEN, Stephan K.**
**2100 Copenhagen (DK)**
- **PITTELKOW, Michael**
**2100 Copenhagen (DK)**
- **KAMOUNAH, Fadhil S.**
**2100 Copenhagen (DK)**

(74) Representative: **BASF IP Association**
**BASF SE**
**GBI - Z078**
**67056 Ludwigshafen (DE)**

(56) References cited:
**SU-A1- 1 728 218 US-B2- 8 454 940**

- **ALEXANDER J FATIADIT: "Pseudo-Oxocarbons. Synthesis of 2, 1 ,3-Bis-, and 1, 2, 3-Tris (Dicyanomethylene) Croconate Salts. New Bond-Delocalized Dianions, "Croconate Violet" and "Croconate Blue"*", JOURNAL OF RESEARCH OF THE NATIONAL BUREAU OF STANDARDS VOLUME, 1 January 1980 (1980-01-01), XP055747835, Retrieved from the Internet <URL:https://nvlpubs.nist.gov/nistpubs/jres/85/ jresv85n2p73_A1b.pdf>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 167 938 B1

- **FABRE P-L ET AL: "Spectroelectrochemical behaviour in dimethylformamide of pseudo-oxocarbons dianions derived from the croconate dianion", ELECTROCHIMICA ACTA, ELSEVIER, AMSTERDAM, NL, vol. 45, no. 17, 1 May 2000 (2000-05-01), pages 2697 - 2705, XP004203119, ISSN: 0013-4686, DOI: 10.1016/S0013-4686(00)00324-8**

## Description

The present invention relates to the field of protection against ultraviolet (UV) radiation. In particular, the present invention relates to compounds of formula (I)

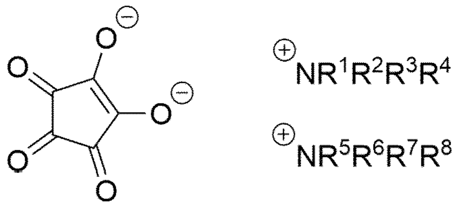

(I)

or a stereoisomer or tautomer thereof,
wherein $R^1$ to $R^8$ are as defined herein. Further, the present invention is concerned with cosmetic or pharmaceutical compositions comprising at least one compound of formula (I). The present invention also relates to compounds of formula (I) and compositions comprising at least one compound of formula (I) for use to protect skin against UV radiations (e.g. in a sunscreen). Further, the present invention is concerned with the use of a compound of formula (I) in body-care products or household cleaning and treating agents as UV filter.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photo allergenic reactions on the skin and can accelerate skin aging.

To protect the human skin from UV radiation, various sun protecting UV filters (also referred to as UV absorbers) exist including UV-A filter, UV-B filter, and broadband filters. These filters are added to e.g. sunscreen or cosmetic compositions. The UV filters are either organic or inorganic, particulate or non-particulate compounds, of which all have a high absorption efficacy in the UV-light range. In general, UV light can be divided into UV-A radiation (320 - 400 nm) and UV-B radiation (280 - 320 nm). Depending on the position of the absorption maxima, UV filters are divided into UV-A and UV-B filters. In case an UV filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

However, the UV filters known in the prior art, which are used in e.g. sunscreen or cosmetic compositions have certain disadvantages. In particular, it is referred to the disadvantage that the vast majority of cosmetic registered organic UV filters are resistant to environmental degradation through chemical, biological, and photolytic processes. Because of their persistence, they bioaccumulate with potential adverse impact on human health and the environment. Due to their wide release into the environment, and especially in the oceans, the demand for biodegradable UV filters is growing.

Currently, the only two biodegradable UV filters (octyl methoxy cinnamates or ethylhexyl methoxycinnamate - OMC and ethylhexyl salicylate - EHS) are both UV-B absorbers. Therefore, it is not possible to prepare biodegradable formulations able to protect e.g. the skin on the whole UV range including the harmful UV-A rays which are responsible for skin photoaging and cancer.

Croconic acid was found to be an excellent UV-A absorber and readily biodegradable. Croconic acid is also known as 4,5-dihydroxy-4-cyclopentene-1,2,3-trione or 4,5-dihydroxycyclopent-4-ene-1,2,3-trione can be expressed by formula (CA).

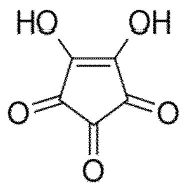

(CA)

The spectroelectrochemical behavior of the di-potassium salt of croconic acid is disclosed in Faber et al., Electrochimica Acta, 2000, 45(17), 2697-2705.

The pH of cosmetic or pharmaceutical products desired for everyday application must be compatible with the physiological skin pH which is 3.5 to 5.5. The pH of cosmetic or pharmaceutical compositions usually is adjusted to pH 4.5 to 7.5. Water soluble salts of croconic acid are compatible with the physiological skin pH and can be easily incorporated in

typical cosmetic or pharmaceutical compositions.

After applying cosmetic or pharmaceutical compositions (e.g. an emulsion) to the skin, water start to evaporate. Evaporation changes the composition and structure of the cosmetic or pharmaceutical composition (e.g. emulsion) on the skin surface. If exemplarily the UV absorber is dissolved in the water phase, water evaporation can significantly affect the amount of UV absorber remaining in the dissolved state (Baki et al., Introduction to Cosmetic Formulation and Technology, John Wiley & Sons, 2015, p. 244).

The sun protection is negatively impacted by the crystallization of croconic acid salts. In addition, croconic acid salts are known to be photo-instable, i.e. are known undergoing photooxidation (Fabre et al., Can. J. Chem., 1995, 73, 1298-1304).

Hence, there is an ongoing need for effective biodegradable UV filters. In particular, it was an object of the present invention to provide an effective biodegradable UV-A filter. In this connection it was a further object to provide biodegradable cosmetic or pharmaceutical compositions comprising ingredients suitable for the whole UV range, in particular wherein the cosmetic or pharmaceutical composition provides for an improved photostability. It was yet another object of the present invention to provide a UV filter (in particular a UV-A filter) which is suitable to use in cosmetic or pharmaceutical compositions, body-care products and/or household cleaning and treating agents.

It has surprisingly been found that at least one of these objects can be achieved by the compound of formula (I) and/or composition according to the present invention.

In particular, the inventors of the present invention have found compounds that effectively absorb light in the UV range relevant for UV protection and which remain dissolved (i.e. do not crystallize) after evaporation of water from aqueous solutions. These compounds are croconic acid salts as defined hereinafter.

In a first aspect, the present invention therefore relates to a compound of formula (I)

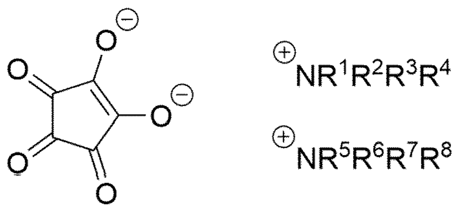

(I)

or a stereoisomer or tautomer thereof,

wherein

$R^1$ is H or $C_1$-$C_4$-alkyl;

$R^2$ is H, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH;

$R^3$ and $R^4$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; or together with the nitrogen to which they are bonded form a 5-membered saturated, partially or fully unsaturated, or aromatic heterocyclic ring, wherein said heterocyclic ring comprises one or more, same or different heteroatoms selected from O, N, or S, wherein said N- and/or S-atoms are independently oxidized or non-oxidized, and wherein each substitutable carbon or heteroatom in the heterocyclic ring is independently unsubstituted or substituted with one or more, same or different substituents $R^M$; or together with the nitrogen to which they are bonded form a double bond to a carbon atom, which is further substituted with two N-atoms, which are independently unsubstituted or substituted with one or more, same or different substituents $R^N$;

$R^5$ is H or $C_1$-$C_4$-alkyl;

$R^6$ is H, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH;

$R^7$ and $R^8$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; or together with the nitrogen to which they are bonded form a 5-membered saturated, partially or fully unsaturated, or aromatic heterocyclic ring, wherein said heterocyclic ring comprises one or more, same or different heteroatoms selected from O, N, or S, wherein said N- and/or S-atoms are independently oxidized or non-oxidized, and wherein each substitutable carbon or heteroatom in the heterocyclic ring is independently unsubstituted or substituted with one or more, same or different substituents $R^M$; or together with the nitrogen to which they are bonded form a double bond to a carbon atom, which is further substituted with two N-atoms, which are independently unsubstituted or substituted with one or more, same or different substituents $R^N$;

$R^M$ is halogen, CN, $NO_2$, $NH_2$, OH, $C_1$-$C_2$-alkyl, $C_1$-$C_2$-alkoxy, C(=O)$R^X$, or two $R^M$ form =O;

$R^N$ is $C_1$-$C_8$-alkyl, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH, wherein each substitutable carbon atom is independently unsubstituted or substituted with one or more, same or different substituents $R^Y$;

$R^X$ is H, $C_1$-$C_2$-alkyl, phenyl, or benzyl;
$R^Y$ is halogen, CN, $NO_2$, $NH_2$, OH, C(=O)$R^X$, or two $R^Y$ form =O; and
n is an integer from 1 to 10.

In the following, preferred embodiments of the substituents in the above formula (I) are described in further detail. It is to be understood that each preferred embodiment is relevant on its own as well as in combination with other preferred embodiments. Furthermore, it is to be understood that the preference in each case also apply to the stereoisomer or tautomer of the compound of the invention.

In a preferred embodiment A1 of the first aspect, $R^1$ and $R^5$ are H.

In a preferred embodiment A2 of the first aspect, $R^2$ to $R^4$ are independently $C_1$-$C_6$-hydroxyalkyl and $R^6$ to $R^8$ are independently $C_1$-$C_6$-hydroxyalkyl.

In a preferred embodiment A3 of the first aspect, $R^1$ and $R^5$ are the same, preferably H; $R^2$ to $R^4$ are the same; and $R^6$ to $R^8$ are the same.

In a preferred embodiment A4 of the first aspect, $R^2$ to $R^4$ and $R^6$ to $R^8$ are hydroxyethyl.

In a preferred embodiment A5 of the first aspect, $R^1$, $R^2$, $R^5$, and $R^6$ are the same;

$R^3$ and $R^4$ together with the nitrogen to which they are bonded form a double bond to a carbon atom, which is further substituted with two N-atoms, which are independently unsubstituted or substituted with one or more, same or different substituents $R^N$;
$R^7$ and $R^8$ together with the nitrogen to which they are bonded form a double bond to a carbon atom, which is further substituted with two N-atoms, which are independently unsubstituted or substituted with one or more, same or different substituents $R^N$;
$R^N$ is $C_1$-$C_6$-alkyl, wherein each substitutable carbon atom is independently unsubstituted or substituted with one or more, same or different substituents $R^Y$: and
$R^Y$ is $NH_2$. OH. or two $R^Y$ form =O.

In a preferred embodiment A6 of the first aspect, $R^1$, $R^2$, $R^5$, and $R^6$ are H;

$R^3$ and $R^4$ together with the nitrogen to which they are bonded form a double bond to a carbon atom, which is further substituted with two N-atoms, wherein one of these two N-atoms is substituted with $R^N$;
$R^7$ and $R^8$ together with the nitrogen to which they are bonded form a double bond to a carbon atom, which is further substituted with two N-atoms, wherein one of these two N-atoms is substituted with $R^N$;
$R^N$ is $C_2$-$C_5$-alkyl, wherein each substitutable carbon atom is independently unsubstituted or substituted with one or more, same or different substituents $R^Y$; and
$R^Y$ is $NH_2$, OH, or two $R^Y$ form =O.

In a preferred embodiment A7 of the first aspect, $R^1$, $R^2$, $R^5$, and $R^6$ are H;

$R^3$ and $R^4$ together with the nitrogen to which they are bonded form a double bond to a carbon atom, which is further substituted with two $NH_2$; and
$R^7$ and $R^8$ together with the nitrogen to which they are bonded form a double bond to a carbon atom, which is further substituted with two $NH_2$.

In a preferred embodiment A8 of the first aspect, the compound according to formula (I) is selected from the group consisting of 4,5-dihydroxy-4-cyclopentene-1,2,3-trione di-triethanolammonium salt, 4,5-dihydroxy-4-cyclopentene-1,2,3-trione di-guanidinium salt, and 4,5-dihydroxy-4-cyclopentene-1,2,3-trione di-argininium salt.

In a preferred embodiment A9 of the first aspect, the compound of formula (I) is selected from the group consisting of

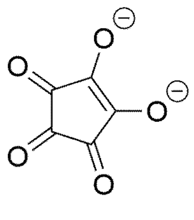 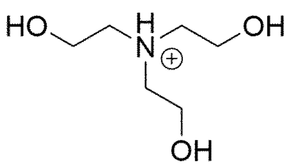 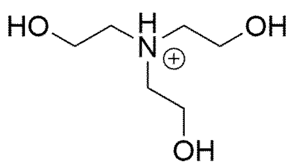 (IIa),

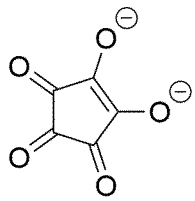 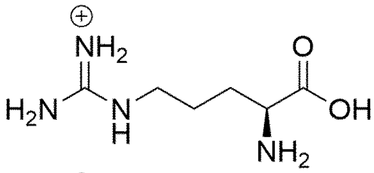 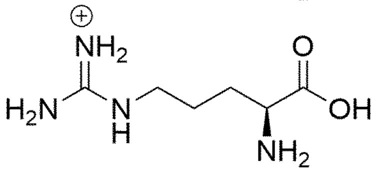 (IIb),

and

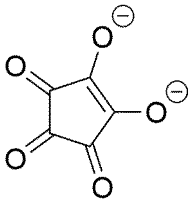 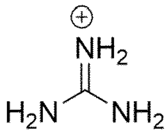 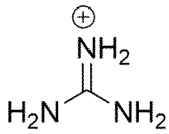 (IIc).

In a second aspect, the present invention relates to a cosmetic or pharmaceutical composition, comprising a compound according to any one of claims 1 to 10 and optionally a dermatologically acceptable emulsifier, thickener, or emollient.

In a preferred embodiment B1 of the second aspect, the cosmetic or pharmaceutical composition further comprises at least one additional UV filter, different to the compound of formula (I), preferably wherein the at least one additional UV filter is a natural and/or biodegradable UV filter.

In a preferred embodiment B2 of the second aspect, the cosmetic or pharmaceutical composition further comprises a photostabilizer, preferably a quencher, wherein preferably the photostabilizer is comprised in the cosmetic or pharmaceutical composition in an amount of at least 0.5 wt.-%, based on the total weight of the cosmetic or pharmaceutical composition.

In a third aspect, the present invention relates to the at least one compound of formula (I) as defined in the first aspect (including all embodiments thereof as described herein) or the cosmetic or pharmaceutical composition as defined in the second aspect (including all embodiments thereof as described herein) for use to protect skin against UV radiations.

In a fourth aspect, the present invention relates to the use of a compound of formula (I) as defined in the first aspect (including all embodiments thereof as described herein) in body-care products or household cleaning and treating agents as UV filter.

In a fifth aspect, the present invention relates to the at least one compound as defined in the first aspect or the cosmetic or pharmaceutical composition as defined in the second aspect for use to protect skin against UV radiations.

Figure legend

Fig. 1A shows the different behavior of biodegradation (readily biodegradable, slowly biodegradable, moderately biodegradable, and non-biodegradable).

Fig. 1B shows the biodegradation behavior of croconic acid, Myritol 318, and Cetiol AB.

Fig. 2 shows the absorption of croconic acid salts in a formulation.

Fig. 3 shows the photostability test of TEA croconic acid salt in a formulation.

Fig. 4 shows the comparison of photostability of a formulation comprising TEA croconic acid salt and a formulation comprising TEA croconic acid salt and a quencher.

Detailed Description

Before describing in detail exemplary embodiments of the present invention, definitions important for understanding the present invention are given.

As used in this specification and in the appended claims, the singular forms of "a" and "an" also include the

respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The term "compound(s) according to the invention", or "compounds of formula (I)" comprises the compound(s) as defined herein as well as a stereoisomer or tautomer thereof.

Depending on the substitution pattern, the compounds according to the invention may have one or more centers of chirality. The invention provides both the single pure enantiomers or pure diastereomers of the compounds according to the invention, and their mixtures and the use according to the invention of the pure enantiomers or pure diastereomers of the compounds according to the invention or their mixtures. Suitable compounds according to the invention also include all possible geometrical stereoisomers (cis/trans isomers or E/Z isomers) and mixtures thereof. Cis/trans isomers may e.g. be present with respect to an amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula (I), i.e. to single enantiomers or diastereomers, as well as to mixtures thereof.

The compounds of formula (I) may be amorphous or may exist in one or more different crystalline states (polymorphs) which may have different macroscopic properties such as stability or show different biological properties such as activities. The present invention relates to amorphous and crystalline compounds of formula (I), mixtures of different crystalline states of the respective compound of formula (I).

Tautomers may be formed, if a substituent is present at the compound of formula (I), which allows for the formation of tautomers such as keto-enol tautomers or the like.

The organic moieties mentioned in the above definitions of the variables are - like the term halogen - collective terms for individual listings of the individual group members. The prefix $C_n$-$C_m$ indicates in each case the possible number of carbon atoms in the group.

The term "halogen" denotes in each case fluorine, bromine, chlorine, or iodine, in particular fluorine, chlorine, or bromine.

The term "alkyl" as used herein denotes in each case a straight-chain or branched alkyl group having usually from 1 to 8 carbon atoms, preferably from 1 to 4 carbon atoms. Examples of an alkyl group are methyl, ethyl, n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, 1,1-dimethylpropyl, and 1,2-dimethylpropyl. Methyl, ethyl, n-propyl, iso-propyl, and iso-butyl, are particularly preferred.

The term "alkoxy" as used herein denotes in each case a straight-chain or branched alkyl group which is bonded via an oxygen atom and has usually from 1 to 6 carbon atoms, preferably 1 to 2 carbon atoms, more preferably 1 carbon atom. Examples of an alkoxy group are methoxy, ethoxy, n-propoxy, iso-propoxy, n-butyloxy, 2-butyloxy, iso-butyloxy, tert.-butyloxy, and the like.

The term "hydroxyalkyl" as used herein denotes in each case a straight-chain or branched alkyl group having usually from 1 to 8 carbon atoms, preferably from 1 to 6 carbon atoms and being further substituted with 1 to 5, preferably with 1 to 2 hydroxy groups, in particular with 1 hydroxy group. Preferably, the one hydroxy group is terminating the straight-chain or branched alkyl group so that the hydroxy group is bonded to an alkyl bridge, which is bonded to the remainder of the molecule. Examples of an hydroxyalkyl group are hydroxymethyl, hydroxyethyl, n-hydroxypropyl, 2-hydroxypropyl, n-hydroxybutyl, 2-hydroxybutyl, 2-hydroxy-2-methylpropyl, n-hydroxypentyl, and n-hydroxyhexyl. Hydroxymethyl, hydroxyethyl, hydroxypropyl, and hydroxybutyl, are preferred, in particular hydroxyethyl.

The term "hydroxyalkenyl" as used herein denotes in each case an unsaturated hydrocarbon group having

usually 2 to 6, preferably 2 to 4 carbon atoms comprising at least one carbon-carbon double bond in any position and being further substituted with 1 to 5, preferably with 1 to 2 hydroxy groups, in particular with 1 hydroxy group. Preferably, the one hydroxy group is terminating the an unsaturated hydrocarbon group so that the hydroxy group is bonded to an alkenyl bridge, which is bonded to the remainder of the molecule. Examples of an hydroxyalkenyl are hydroxyvinyl, hydroxyallyl, hydroxymethallyl, hydroxybuten-1-yl, 2-hydroxy-2-penten-1-yl, 1-hydroxy-3-penten-1-yl and the like. If geometric isomers are possible with regard to the double bond, the present invention relates to both, the E- and Z-isomers.

The term "aminoalkyl" as used herein denotes in each case a straight-chain or branched alkyl group having usually from 1 to 8 carbon atoms, preferably from 1 to 6 carbon atoms and being further substituted with 1 to 5, preferably with 1 to 2 amino groups, in particular 1 amino group. Preferably, the one amino group is terminating the straight-chain or branched alkyl group so that the amino group is bonded to an alkyl bridge, which is bonded to the remainder of the molecule. Examples of an aminoalkyl group are aminomethyl, aminoethyl, n-aminopropyl, 2-aminopropyl, n-aminobutyl, 2-aminobutyl, 2-amino-2-methylpropyl, n-aminopentyl, and n-aminohexyl. Aminomethyl, aminoethyl, aminopropyl, and aminobutyl, are preferred, in particular aminoethyl.

The term "($C_n$-$C_m$-alkyl)" as used herein denotes in each case a linker moiety, wherein the thereto attached moieties are attached to the terminal carbons.

The term "C(=O)" as used therein denotes in each case a carbonyl moiety.

The term "C(=O)-($C_n$-$C_m$-alkyl)" as used herein denotes in each case an alkylcarbonyl, referring to a straight-chain or branched alkyl group as defined above, which is bonded via the carbon atom of a carbonyl group C(=O) to the remainder of the molecule.

The term "aryl" or "aromatic carbocycle" preferably includes 6-membered aromatic carbocyclic rings based on carbon atoms as ring members. A preferred example is phenyl.

The term "heterocyclic" or "heterocyclyl" includes, unless otherwise indicated, in general a 3-to 9-membered, preferably a 4- to 8-membered or 5- to 7-membered, more preferably 5- or 6-membered, in particular 6-membered monocyclic ring. The heterocycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. The heterocycle typically comprises one or more, e.g. 1, 2, 3, or 4, preferably 1, 2, or 3 heteroatoms selected from N, O and S as ring members, where S-atoms as ring members may be present as S, SO or $SO_2$. The remaining ring members are carbon atoms. In a preferred embodiment, the heterocycle is an aromatic heterocycle, preferably a 5- or 6-membered aromatic heterocycle comprising one or more, e.g. 1, 2, 3, or 4, preferably 1, 2, or 3 heteroatoms selected from N, O and S as ring members, where S-atoms as ring members may be present as S, SO or $SO_2$. Examples of aromatic heterocycles are provided below in connection with the definition of "hetaryl". "Hetaryls" or "heteroaryls" are covered by the term "heterocycles". The saturated or partially or fully unsaturated heterocycles usually comprise 1, 2, 3, 4 or 5, preferably 1, 2 or 3 heteroatoms selected from N, O and S as ring members, where S-atoms as ring members may be present as S, SO or $SO_2$. The skilled person is aware that S, SO or $SO_2$ is to be understood as follows:

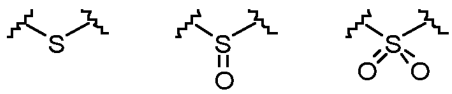

Further, a skilled person is aware that resonance structures of the oxidized forms may be possible. Saturated heterocycles include, unless otherwise indicated, in general 3- to 9-membered, preferably 4- to 8-membered or 5- to 7-membered, more preferably 5- or 6-membered monocyclic rings comprising 3 to 9, preferably 4 to 8 or 5 to 7, more preferably 5 or 6 atoms comprising at least one heteroatom, such as pyrrolidine, tetrahydrothiophene, tetrahydrofuran, piperidine, tetrahydropyran, dioxane, morpholine or piperazine.

The term "hetaryl" or "heteroaryl" or "aromatic heterocycle" or "aromatic heterocyclic ring" includes monocyclic 5- or 6-membered aromatic heterocycles comprising as ring members 1, 2, 3 or 4 heteroatoms selected from N, O and S, where S-atoms as ring members may be present as S, SO or $SO_2$. Examples of 5- or 6-membered aromatic heterocycles include pyridyl (also referred to as pyridinyl), i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl.

When referring to compositions and the weight percent of the therein comprised ingredients it is to be understood that according to the present invention the overall amount of ingredients does not exceed 100% (± 1% due to rounding).

The term "body-care product" refers to any product suitable to apply to the human body, e.g. sunscreen

compositions, bath and shower products, preparations containing fragrances and odoriferous substances, hair-care products, dentifrices, decorative preparations, and cosmetic formulations containing active ingredients. Preferred are sunscreen compositions.

The term "sunscreen composition" or "sunscreen" or "skin-care product" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic UV filters, as well as other ingredients or additives, e.g., emulsifiers, emollients, viscosity regulators, stabilizers, preservatives, or fragrances.

The term "pharmaceutical composition" refers to a composition containing at least one active ingredient. Suitable pharmaceutical compositions are hormone preparations, vitamin preparations, vegetable extract preparations, and antibacterial preparations.

Suitable bath and shower additives are, e.g., shower gels, bath-salts, bubble baths and soaps. Suitable preparations containing fragrances and odoriferous substances are in particular scents, perfumes, toilet waters and shaving lotions (aftershave preparations).

Suitable hair-care products are, e.g., shampoos for humans and animals, in particular dogs, hair conditioners, products for styling and treating hair, perming agents, hair sprays and lacquers, hair gels, hair fixatives and hair dyeing or bleaching agents.

Suitable dentifrices are, e.g., tooth creams, toothpastes, mouth-washes, mouth rinses, antiplaque preparations and cleaning agents for dentures.

Suitable decorative preparations are, e.g., lipsticks, nail varnishes, eye shadows, mascaras, dry and moist make-up, rouge, powders, depilatory agents and suntan lotions.

Suitable cosmetic formulations containing active ingredients are, e.g., hormone preparations, vitamin preparations, vegetable extract preparations and antibacterial preparations.

The cited body-care products can be in the form of creams, ointments, pastes, foams, gels, lotions, powders, make-ups, sprays, sticks or aerosols. They preferably contain the compound of formula (I) in the aqueous phase.

The term "light stabilizers" as used herein are compounds suitable for protecting body-care and household cleaning and treating agents against photolytic degradation. The light stabilizer is usually present in the body-care product in a concentration of 50 to 1000 ppm.

The term "household cleaning and treating agent" refers to any product suitable to clean or treat household objects, e.g., liquid scouring agents, glass detergents, neutral cleaners (all-purpose cleaners), acid household cleaners (bath), WC cleaners, preferably in washing, rinsing and dishwashing agents, clear rinsing agents, dishwasher detergents, shoe polishes, polishing waxes, floor detergents and polishes, metal, glass and ceramic cleaners, textile-care products, agents for removing rust, color and stains (stain remover salt), furniture and multipurpose polishes and leather dressing agents (leather sprays).

Preferably, household cleaning agents are aqueous or alcoholic (e.g. ethanol or isopropyl alcohol) solutions of one or more of the following components: -anionic, nonionic, amphoteric and/or cationic surfactants -soaps, prepared by saponification of animal and vegetable greases -organic acids, like hydrochloric acid, phosphoric acid, or sulfuric acid, -for basic products inorganic (NaOH or KOH) or organic bases; -abrasives for improved cleaning of surfaces, - waxes and/or silicones for maintenance and protection of surfaces, -polyphosphates, - substances which eliminate hypochlorite or halogens; -peroxides comprising bleaching activators like TAED, for example sodium perborate or $H_2O_2$; -enzymes; -in washing detergents discoloration inhibitors, soil-release compounds, grey scale inhibitors, foam inhibitors, fluorescent whitening agents; -cleaning agents based on wax may comprise solvents selected from benzine, turpentine and/or paraffines and emulsifiers based on wax; -filling agents like silicates, polyphosphates, Zeolithes for powdery cleaning agents; -pigments, lakes or soluble dyes; -perfumes; and -light stabilizers, antioxidants and chelating agents.

The term "photostability" refers to the ability of a UV filter or any other molecule, which is exposed to sunlight, to stay stable upon irradiation. In particular, this means that the compound does not undergo a degradation process upon UV radiation.

The term "sun protection factor (SPF)" as used herein indicates how well the skin is protected by a sunscreen composition. In particular, the factor indicates how much longer the protected skin may be exposed to the sun without getting a sunburn in comparison to untreated skin. For example, if a sunscreen composition with an SPF of 15 is evenly applied to the skin of a person usually getting a sunburn after 10 minutes in the sun, the sunscreen allows the skilled person to stay in the sun 15 times longer. In other words, SPF 15 means that 1/15 of the burning UV radiation will reach the skin, assuming sunscreen is applied evenly at a thick dosage of 2 milligrams per square centimeter ($mg/cm^2$).

The term "critical wavelength" is defined as the wavelength at which the area under the UV protection curve (% protection versus wavelength) represents 90 % of the total area under the curve in the UV region (280-400 nm). For example, a critical wavelength of 370 nm indicates that the protection of the sunscreen composition is not limited to the

wavelengths of UV-B, i.e. wavelengths from 280-320 nm, but extends to 370 nm in such a way that 90 % of the total area under the protective curve in the UV region are reached at 370 nm.

The term "ultraviolet filter" or "UV filter" as used herein refers to organic or inorganic compounds, which can absorb and/or reflect UV radiation caused by sunlight. UV filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UV-A protection factor, which is at least one third of the labelled sun protection factor (SPF), e.g. if the sunscreen composition has an SPF of 30 the UV-A protection factor has to be at least 10.

The term "biodegradation" as used herein denotes that microorganism metabolize the material completely to $CO_2$, energy, water, and biomass in an aerobic process (according to OECD guideline).

Preferred embodiment regarding the compound of formula (I) as well as the use of the compound of formula (I) to protect skin against UV radiations or as a light stabilizer are described hereinafter. It is to be understood that the preferred embodiments of the invention are preferred alone or in combination with each other. Further, preferred embodiments regarding the cosmetic or pharmaceutical composition comprising a compound of formula (I) as well as the use of such a cosmetic or pharmaceutical composition e.g. in sunscreens are described hereinafter.

As indicated above, the present invention relates in one embodiment to a compound of formula (I)

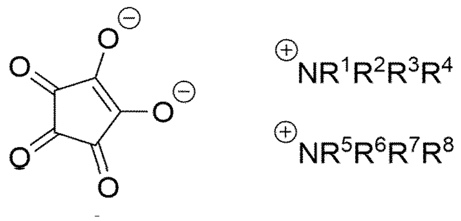

(I)

or a stereoisomer or tautomer thereof,
wherein

$R^1$ is H or $C_1$-$C_4$-alkyl;

$R^2$ is H, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH;

$R^3$ and $R^4$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; or together with the nitrogen to which they are bonded form a 5-membered saturated, partially or fully unsaturated, or aromatic heterocyclic ring, wherein said heterocyclic ring comprises one or more, same or different heteroatoms selected from O, N, or S, wherein said N- and/or S-atoms are independently oxidized or non-oxidized, and wherein each substitutable carbon or heteroatom in the heterocyclic ring is independently unsubstituted or substituted with one or more, same or different substituents $R^M$; or together with the nitrogen to which they are bonded form a double bond to a carbon atom, which is further substituted with two N-atoms, which are independently unsubstituted or substituted with one or more, same or different substituents $R^N$;

$R^5$ is H or $C_1$-$C_4$-alkyl;

$R^6$ is H, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH;

$R^7$ and $R^8$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; or together with the nitrogen to which they are bonded form a 5-membered saturated, partially or fully unsaturated, or aromatic heterocyclic ring, wherein said heterocyclic ring comprises one or more, same or different heteroatoms selected from O, N, or S, wherein said N- and/or S-atoms are independently oxidized or non-oxidized, and wherein each substitutable carbon or heteroatom in the heterocyclic ring is independently unsubstituted or substituted with one or more, same or different substituents $R^M$; or together with the nitrogen to which they are bonded form a double bond to a carbon atom, which is further substituted with two N-atoms, which are independently unsubstituted or substituted with one or more, same or different substituents $R^N$;

$R^M$ is halogen, CN, $NO_2$, $NH_2$, OH, $C_1$-$C_2$-alkyl, $C_1$-$C_2$-alkoxy, C(=O)$R^X$, or two $R^M$ form =O;

$R^N$ is $C_1$-$C_8$-alkyl, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH, wherein each substitutable carbon atom is independently unsubstituted or substituted with one or more, same or different substituents $R^Y$;

$R^X$ is H, $C_1$-$C_2$-alkyl, phenyl, or benzyl;

$R^Y$ is halogen, CN, $NO_2$, $NH_2$, OH, C(=O)$R^X$, or two $R^Y$ form =O; and
n is an integer from 1 to 10.

It was surprisingly found that the compounds according to the present invention especially benefit from specific advantageous in body-care products (e.g. sunscreen) due to their deliquescent behavior. In this connection, it is to be understood that deliquescent behavior denotes that crystalline substances form a solution when the ambient relative humidity (RH) reaches a certain threshold value.

There are several types of water solid interaction. One type of solid water inaction that is of great importance for highly water soluble crystalline compounds is the phenomenon of deliquescence.

Deliquescence is a first order phase transformation of the solid to a saturated solution which is triggered at a well defined relative humidity which depends on the properties of the solid and the temperature. When this RH is reached, the aqueous solution is the thermodynamically favored phase and dissolution commences. Below this RH, the crystalline solid surrounded by gaseous water is favorable (Mauer et al., Pharmaceutical Development and Technology, 2010, vol. 15, 6, p. 582-594).

After evaporation of water from solutions of the compounds of formula (I) at pH 7 $\pm$ 1, the compounds begin to crystallize. In general, water evaporation retardants can be used to slow down the water's evaporation rate as much as possible. However, following this approach there is still a high risk, that the compounds precipitates when water is evaporated.

Compositions, comprising the compounds according to the present invention (pH 7 $\pm$ 1), the compounds according to the present invention remain dissolved after evaporation of water from aqueous solutions e.g. from the water phase of a cosmetic or pharmaceutical composition such as an emulsion.

Preferred embodiments regarding the compounds of formula (I), which are relevant for all aspects of the invention, are defined hereinafter.

In one embodiment of the present invention, $R^1$ and $R^5$ are the same, preferably are H.

In one preferred embodiment of the present invention, $R^3$, $R^4$, $R^7$, and $R^8$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH. Preferrably, $R^3$, $R^4$, $R^7$, and $R^8$ are independently $C_1$-$C_6$-hydroxyalkyl, $C_2$-$C_4$-hydroxyalkenyl, or $C_1$-$C_6$-aminoalkyl.

In one preferred embodiment of the present invention, $R^1$ and $R^5$ are the same, preferably are H; and $R^2$ to $R^4$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; and $R^8$ to $R^8$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH.

In one embodiment of the present invention, $R^2$ to $R^4$ are independently $C_1$-$C_6$-hydroxyalkyl; and $R^6$ to $R^8$ are independently $C_1$-$C_6$-hydroxyalkyl. In a preferred embodiment, $R^2$ to $R^4$ are independently $C_1$-$C_5$-hydroxyalkyl, more preferably $C_1$-$C_4$-hydroxyalkyl, and in particular $C_1$-$C_3$-hydroxyalkyl; and $R^6$ to $R^8$ are independently $C_1$-$C_5$-hydroxyalkyl, more preferably $C_1$-$C_4$-hydroxyalkyl, and in particular $C_1$-$C_3$-hydroxyalkyl.

In one embodiment of the present invention, $R^1$ and $R^5$ are the same, preferably H; $R^2$ to $R^4$ are the same; and $R^6$ to $R^8$ are the same.

In one embodiment of the present invention, $R^2$ to $R^4$ and $R^6$ to $R^8$ are $C_2$-$C_4$-hydroxyalkene, $C_1$-$C_6$-aminoalkyl, $(CH_2O)_n$-OH, $(CH_2CH_2O)_n$-OH, or $(CH_2CH_2CH_2O)_n$-OH. In this connection, it is preferred that $R^1$ and $R^5$ are H.

In one embodiment of the present invention, $R^2$ to $R^4$ and $R^6$ to $R^8$ are hydroxyethyl. In another embodiment of the present invention, $R^2$ to $R^4$ and $R^6$ to $R^8$ are n-hydroxypropyl, n-hydroxybutyl, n-hydroxypentyl, or n-hydroxyhexyl. In this connection, it is preferred that $R^1$ and $R^5$ are H.

In a preferred embodiment of the present invention, $R^1$ and $R^5$ are H; and $R^2$ to $R^4$ and $R^6$ to $R^8$ are hydroxyethyl.

In one embodiment of the present invention, $R^1$, $R^2$, $R^5$, and $R^6$ are the same;

$R^3$ and $R^4$ together with the nitrogen to which they are bonded form a double bond to a carbon atom, which is further substituted with two N-atoms, which are independently unsubstituted or substituted with one or more, same or different substituents $R^N$;
$R^7$ and $R^8$ together with the nitrogen to which they are bonded form a double bond to a carbon atom, which is further substituted with two N-atoms, which are independently unsubstituted or substituted with one or more, same or different substituents $R^N$;
$R^N$ is $C_1$-$C_6$-alkyl, wherein each substitutable carbon atom is independently unsubstituted or substituted with one or more, same or different substituents $R^Y$; and
$R^Y$ is $NH_2$, OH, or two $R^Y$ form =O. In this connection it is preferred that $R^1$, $R^2$, $R^5$, and $R^6$ are H.

In one embodiment of the present invention, $R^1$, $R^2$, $R^5$, and $R^6$ are H;

$R^3$ and $R^4$ together with the nitrogen to which they are bonded form a double bond to a carbon atom, which is further

substituted with two N-atoms, wherein one of these two N-atoms is substituted with $R^N$;
$R^7$ and $R^8$ together with the nitrogen to which they are bonded form a double bond to a carbon atom, which is further substituted with two N-atoms, wherein one of these two N-atoms is substituted with $R^N$;
$R^N$ is $C_2$-$C_5$-alkyl, wherein each substitutable carbon atom is independently unsubstituted or substituted with one or more, same or different substituents $R^Y$; and
$R^Y$ is $NH_2$, OH, or two $R^Y$ form =O.

In a preferred embodiment of the present invention, $R^1$, $R^2$, $R^5$, and $R^6$ are H;

$R^3$ and $R^4$ together with the nitrogen to which they are bonded form a double bond to a carbon atom, which is further substituted with two N-atoms, wherein one of these two N-atoms is substituted with $R^N$;
$R^7$ and $R^8$ together with the nitrogen to which they are bonded form a double bond to a carbon atom, which is further substituted with two N-atoms, wherein one of these two N-atoms is substituted with $R^N$;
$R^N$ is $C_5$-alkyl, wherein each substitutable carbon atom is independently unsubstituted or substituted with one or more, same or different substituents $R^Y$; and
$R^Y$ is $NH_2$, OH, or two $R^Y$ form =O.

In one embodiment of the present invention, $R^1$, $R^2$, $R^5$, and $R^6$ are H;

$R^3$ and $R^4$ together with the nitrogen to which they are bonded form a double bond to a carbon atom, which is further substituted with two $NH_2$; and
$R^7$ and $R^8$ together with the nitrogen to which they are bonded form a double bond to a carbon atom, which is further substituted with two $NH_2$.

In one embodiment of the present invention, $R^1$, $R^2$, $R^5$, and $R^6$ are H;

$R^3$ and $R^4$ together with the nitrogen to which they are bonded form a double bond to a carbon atom, which is further substituted with two N-atoms, wherein one of these two N-atoms is substituted with $R^N$;
$R^7$ and $R^8$ together with the nitrogen to which they are bonded form a double bond to a carbon atom, which is further substituted with two $NH_2$;
$R^N$ is $C_5$-alkyl, wherein each substitutable carbon atom is independently unsubstituted or substituted with one or more, same or different substituents $R^Y$; and
$R^Y$ is $NH_2$, OH, or two $R^Y$ form =O.

In one embodiment of the present invention, the compound according to formula (I) is selected from the group consisting of 4,5-dihydroxy-4-cyclopentene-1,2,3-trione di-triethanolammonium salt, 4,5-dihydroxy-4-cyclopentene-1,2,3-trione di-guanidinium salt, and 4,5-dihydroxy-4-cyclopentene-1,2,3-trione di-argininium salt.
In this connection, it is noted that e.g. 4,5-dihydroxy-4-cyclopentene-1,2,3-trione di-triethanolammonium salt may also be known as 4-cyclopentene-1,2,3-trione-4,5-dihydroxy compound with triethanolamine or being expressed as $C_5H_2O_5 \cdot 2\ N(CH_2CH_2OH)_3$, further illustrated as follows:

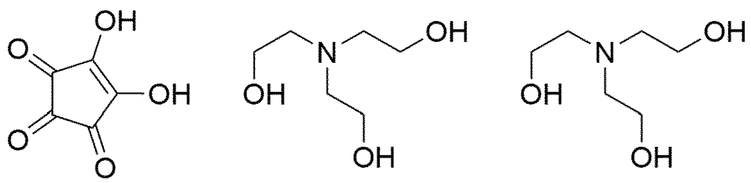

Same possible nomenclature variation applies for 4,5-dihydroxy-4-cyclopentene-1,2,3-trione di-guanidinium salt and 4,5-dihydroxy-4-cyclopentene-1,2,3-trione di-argininium salt.
In one embodiment of the present invention, the compound of formula (I) is selected from the group consisting of

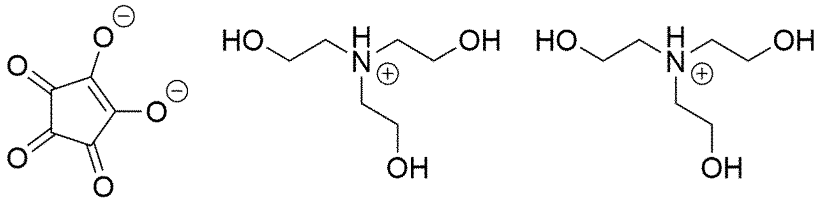

(IIa),

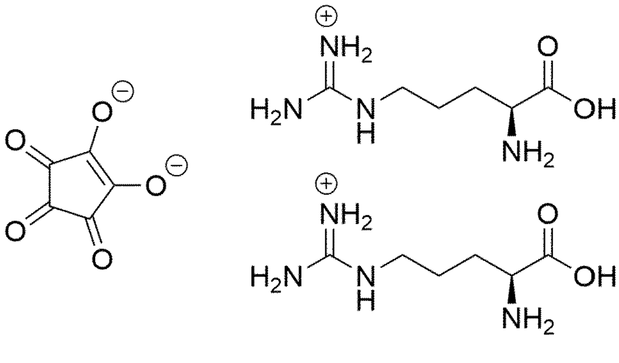

(IIb),

and

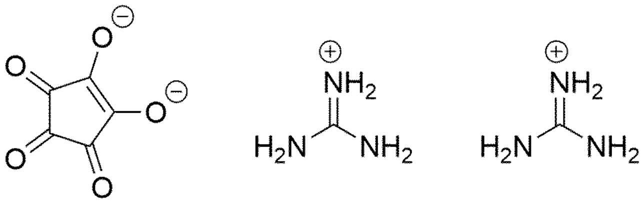

(IIc).

In a particular embodiment of the present invention, the compound of formula (I) is

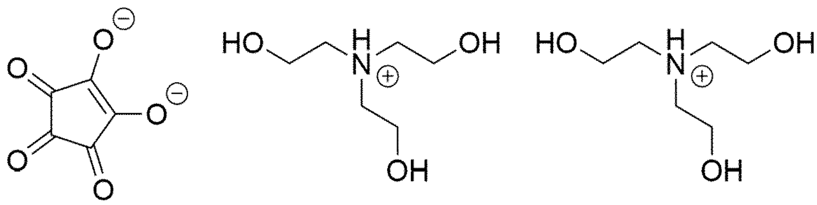

(IIa).

As indicated above, the present invention further relates to cosmetic or pharmaceutical composition, comprising a compound according to the present invention and optionally a dermatologically acceptable emulsifier, thickener, or emollient.

Preferably, the compound of formula (I) is present in the cosmetic or pharmaceutical composition in an amount of from 0.5 to 50 wt.-%, preferably from 1 to 45 wt.-%, more preferably from 2 to 40 wt.-%, and in particular from 3 to 35 wt.-%, based on the total weight of the cosmetic or pharmaceutical composition.

In one embodiment of the present invention, the cosmetic or pharmaceutical composition further comprises at least one additional UV filter, different to the compound of formula (I). The at least one additional UV filter may be an organic or an inorganic UV filter.

Preferably, the at least one additional UV filter is a natural and/or biodegradable UV filter. Thus, in one embodiment of the present invention, the cosmetic or pharmaceutical composition further comprises at least one additional UV filter, different to the compound of formula (I), wherein the at least one additional UV filter is a natural and/or biodegradable UV filter. The at least one additional UV filter may be present in the water or the oil phase. In a preferred embodiment of the present invention, the at least one additional UV filter is present in the oil phase. In connection with this embodiment, the cosmetic or pharmaceutical composition provides an even more improved protection against UV radiation since a UV filter is present in the oil phase and in the water phase, thus providing an improved repartition of the UV filters within the cosmetic or pharmaceutical composition.

In a preferred embodiment, the at least one additional UV filter, different to the compound of formula (I) is a UV-A filter, preferably selected from the group consisting of 2-(4'-diethylamino-2'-hydroxybenzoyl)benzoic acid hexyl ester (also referred to as diethylamino hydroxybenzoyl hexyl benzoate or DHHB), butyl methoxydibenzoylmethane (also known as 4-(tert.-butyl)-4'-methoxydibenzoylmethane or BMDBM), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-( 2-ethylhexyloxymethyl)-4-methyl-phenol, methylene bis-benzotriazolyl tetramethyl butyl phenol, terephthalylidene dicamphor sulfonic acid, and combinations thereof.

In another preferred embodiment, the at least one additional UV filter, different to the compound of formula (I) is a UV-B filter, preferably selected from the group consisting of octinoxate (also referred to as ethylhexyl methoxycinnamate), ethylhexyl salicylate, 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, phenylbenzimidazole sulfonic acid, and combinations thereof.

In another preferred embodiment, the at least one additional UV filter, different to the compound of formula (I) is an inorganic UV filter such as zinc oxide and titanium dioxide.

In yet another preferred embodiment, the at least one additional UV filter, different to the compound of formula (I) is selected from the group consisting of 2-(4'-diethylamino-2'-hydroxybenzoyl)benzoic acid hexyl ester (also referred to as diethylamino hydroxybenzoyl hexyl benzoate or DHHB), butyl methoxydibenzoylmethane (also known as 4-(tert.-butyl)-4'-methoxydibenzoylmethane or BMDBM), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-( 2-ethylhexyloxymethyl)-4-methyl-phenol, methylene bis-benzotriazolyl tetramethyl butyl phenol, terephthalylidene dicamphor sulfonic acid, octinoxate (also referred to as ethylhexyl methoxycinnamate), ethylhexyl salicylate, 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, phenylbenzimidazole sulfonic acid, zinc oxide, titanium dioxide, and combinations thereof.

In one embodiment of the present invention, the cosmetic or pharmaceutical composition further comprises at least two additional UV filter, different to the compound of formula (I).

In one embodiment of the present invention, the cosmetic or pharmaceutical composition according to the present invention further comprising a photostabilizer. Photostabilizers may be used in a quantity sufficient to obtain a substantial and significant improvement in the photostability of the cosmetic or pharmaceutical composition containing compound of formula (I). This minimum quantity of photostabilizer to be used may vary depending on the starting quantity of compound of formula (I) present in the composition and depending on the nature of the dermatologically acceptable support used in the composition. It may be determined without difficulty using a conventional photostability measuring test.

In a preferred embodiment of the present invention, the photostabilizer is present in the cosmetic or pharmaceutical composition in an amount of at least 0.5 wt.-%, preferably at least 1 wt.-%, more preferably at least 2 wt.-%, and in particular at least 4 wt.-%, based on the total weight of the cosmetic or pharmaceutical composition. In another preferred embodiment, the photostabilizer is present in the cosmetic or pharmaceutical composition in an amount of from 0.5 to 15 wt.-%, preferably from 1 to 12 wt.-%, more preferably from 2 to 10 wt.-%, and in particular from 4 to 8 wt.-%, based on the total weight of the cosmetic or pharmaceutical composition.

In a preferred embodiment, the photostabilizer is a quencher. Preferably, the quencher is present in the cosmetic or pharmaceutical composition in an amount of at least 0.5 wt.-%, preferably at least 1 wt.-%, more preferably at least 1.5 wt.-%, and in particular at least 2 wt.-%, based on the total weight of the cosmetic or pharmaceutical composition. In another preferred embodiment, the quencher is present in the cosmetic or pharmaceutical composition in an amount of from 0.5 to 10 wt.-%, preferably from 1 to 8 wt.-%, more preferably from 1.5 to 6 wt.-%, and in particular from 2 to 5 wt.-%, based on the total weight of the cosmetic or pharmaceutical composition.

In a preferred embodiment of the present invention, the photostabilizer is selected from the group consisting of

- CAS-Regno. 444811-29-4, Propanedioic acid, [(4-hydroxy-3,5-dimethoxyphenyl)methylene]-, bis(2-ethylhexyl) ester (Oxynex ST)
- CAS-Regno. 477844-93-2, Octofluorene
- 2-phenylethylbenzoate
- CAS-Regno. 127474-91-3, 2,6-Naphthalenedicarboxylic acid, bis(2-ethylhexyl) ester (Hallbrite TQ, Corapan TQ)
- CAS-Regno. 68890-66-4, Octopirox
- Tinogard TT (INCI Tetradibutyl Pentaerithrityl Hydroxy- hydrocinnamate)
- Tinogard HS (INCI Sodium Benzotriazolyl Butylphenol Sulfonate)
- Tinogard TL (INCI Benzotriazolyl Dodecyl p-Cresol)
- Phenol, 2-(2H-benzotriazol-2-yl)-6-dodecyl-4-methyl-, branched and linear

- Cibafast H Liquid (INCI Sodium Benzotriazolyl Butylphenol Sulfonate, Buteth-3, Tributyl Citrate)
- Tinogard AS (INCI Bumetrizole)
- Tris(tetramethylhydroxypiperidinol) citrate (Tinogard Q)
- Piperidinol, 1-hydroxy-2,2,6,6-tetramethyl-, 2-hydroxy-1,2,3-propanetricarboxylate (3:1) (salt)
- CAS-Regno. 1750-49-8, N-(2-Hydroxypropyl)urea
- CAS-Regno. 2078-71-9, N-(2-Hydroxyethyl)urea
- mixture of n-butylphthalimide and isopropylphthalimide
- CAS-Regno. 872424-70-9
- CAS-Regno. 872424-71-0
- CAS-Regno. 872424-72-1
- CAS-Regno. 872424-73-2
- CAS-Regno.6197-30-4, Octocrylene
- CAS-Regno. 118-60-5, 2-Ethylhexyl salicylate
- CAS-Regno. 180898-37-7, Disodium phenyldibenzimidazoletetrasulfonate Neo Heliopan AP or Neo-Heliopan APC
- CAS-Regno. 302776-68-7, Uvinul A Plus
- CAS-Regno.70356-09-1, 4-tert-Butyl-4'-methoxydibenzoylmethane (Avobenzone)
- Tinosorb M (micronized CAS-Regno. 103597-45-1)
- Tinosorb S (CAS-Regno. 187393-00-6)
- CAS-Regno. 88122-99-0, Ethylhexyl triazone (Octyl triazone; Uvinul T 150)
- CAS-Regno. 154702-15-5, Diethylhexyl butamidotriazone (Uvasorb HEB)
- CAS-Regno. 155633-54-8, Drometrizole trisiloxane (Mexoryl XL), and
- CAS-Regno. 92761-26-7 Mexoryl SX; Terephthalylidene-3,3'-dicamphor-10,10'-disulfonic acid

Preferred are triplet quencher such as Tinogard Q (comprising tris(tetramethylhydroxypiperidinol) citrate (also known as 4-Piperidinol, 1-hydroxy-2,2,6,6-tetramethyl-, 2-hydroxy-1,2,3-propanetricarboxylate (3:1) (salt) or CAS-Regno. 220410-74-2), water (CAS-Regno. 7732-18-5) and ethanol (CAS-Regno. 64-17-5)). Tinogard Q is an Excited State Quencher, water soluble and is delivered in liquid formulation. Excited State Quencher helps to prevent light induced degradation of formulations in transparent packagings. In combination with UV absorbers it results in highly effective stabilizer systems. In general, Tinogard Q is used to protect personal care products from degradation caused by exposure to UV radiation in transparent packaging. It is applied in e.g. body wash, face wash, liquid hand soap, skin care, shampoo, hair conditioner, and fragrances.

As indicated above, in another aspect, the present invention relates in one embodiment to the use of a compound of formula (I) as defined herein (including all embodiments thereof as described herein) or a cosmetic or pharmaceutical composition as defined herein (including all embodiments thereof as described herein) to protect skin against UV radiations. In connection with this embodiment, the compound of formula (I) as defined herein is used for protecting the skin from UV radiation, in particular for protecting human skin from UV radiation. Preferred is the use of a compound of formula (I) as defined herein (including all embodiments thereof as described herein) or a cosmetic or pharmaceutical composition as defined herein (including all embodiments thereof as described herein) in a sunscreen.

As indicated above, in another aspect, the present invention relates in one embodiment to the at least one compound as defined herein (including all embodiments thereof as described herein) or the cosmetic or pharmaceutical composition as defined herein (including all embodiments thereof as described herein) for use to protect skin against UV radiations.

As indicated above, the present invention further relates to the use of a compound of formula (I) as defined herein (including all embodiments thereof as described herein) in body-care products or household cleaning and treating agents as a light stabilizer to protect ingredients against photolytic degradation.

In connection with the above embodiments, it is to be understood that the compounds of formula (I) are in particularly suitable for use as UV filter and hence for protection of the skin from UV radiation.

Furthermore, in connection with the above embodiments, it is to be understood that the body-care product (e.g. a sunscreen composition) may comprise at least one additive.

In one embodiment, the at least one additive is selected from the group consisting of emulsifier, emollients, viscosity regulators (thickeners), sensory enhancers, adjuvants, preservatives, and combinations thereof.

Preferred emulsifiers include

- glucose derivatives such as cetearyl glucoside, arachidyl glucoside, lauryl glucoside, polyglyceryl-3 methylglucose distearate, methyl glucose sesquistearate;
- sucrose derivative such as sucrose polystearate, sucrose palmitate;
- sorbitol derivatives;
- glycerides of fatty acids such as glyceryl stearate, glyceryl oleate;

- glumatic acid derivatives such as sodium stearoyl glutamate;
- sulfosuccinic acid derivatives such as disodium cetearyl sulfosuccinate;
- phosphoric acid derivatives such as potassium cetyl phosphate;
- fatty acid esters of polyglyceryl such as polyglyceryl-3-diisostearate, polyglyceryl-2-dipolyhydroxystearate;
- oxyalkenylated organomodified silicone / polysiloxane / polyalkyl / polyether copolymers and derivatives.

Preferred emollients include

- esters of linear or branched fatty acids with linear or branched fatty alcohols such as propylheptyl caprylate, coco caprylate, isopropyl myristate, ethylhexyl palmitate;
- esters of aromatic carboxylic acids with linear or branched fatty alcohols such as $C_{12}$-$C_{15}$-alkyl benzoate, ethylhexyl benzoate, phenethyl benzoate;
- dicarboxylic acid esters with linear or branched alcohols such as dibutyl adipate, dicaprylyl carbonate, diisopropyl sebacate;
- esters of hydroxycarboxylic acids with linear or branched fatty alcohols;
- esters of linear or branched fatty acids with polyhydric alcohol such as butylene glycol dicaprylate/dicaprate;
- mono-, di-, tri-glycerides based on $C_6$-$C_{18}$ fatty acids such as caprylic / capric triglycerides, coco glycerides;
- guerbet alcohols such as octyldodecynol;
- hydrocarbons such as hydrogenated polyisobutene, mineral oil, squalene, isohexadecane;
- ethers such as dicaprylyl ether;
- silicone derivatives (organomodified polysiloxanes) such as dimethylpolysiloxane, cyclic silicones.

Preferred thickeners include

- fatty alcohols such as cetyl alcohol, cetearyl alcohol, stearyl alcohol;
- fatty acids such as stearic acid;
- fatty acid esters such as myristyl stearate;
- waxes such as beeswax, carnauba wax, microcrystalline wax, ceresin, ozocerite;
- polysaccharides or derivatives such as xanthan gum, guar gum, agar gum, alginates, gellan gum, carraghenan;
- polyacrylates or homopolymers of reticulated acrylic acids or polyacrylamides such as carbomers, acrylate copolymers, acrylate / $C_{10}$-$C_{30}$-alkyl acrylate crosspolymer, acrylate / beheneth-25 methacrylate copolymer;
- silicate derivatives such as magnesium silicates;
- cellulose derivatives such as hydroxypropyl cellulose.

Preferred sensory enhancers include

- polyamide derivatives such as nylon-12;
- polymethyl methacrylates;
- silica;
- mica;
- polymethylsilsesquioxane;
- polyethylene;
- starch derivatives such as aluminum starch octenylsuccinate;
- dimethicone derivatives;
- boron nitride;
- HDI / trimethylol hexyllactone crosspolymer.

Preferred adjuvants include

- tocopherol derivatives;
- retinol derivatives;
- ascorbic acid derivatives;
- bisabolol;
- allantoin;
- panthenol;
- chelating agents (EDTA, EDDS, EGTA, phytic acid, piroctone olamine);
- ethylhexyl glycerin;
- caprylyl glycol;

- hydroxyacetophenone;
- caprylhydroxymic acid;
- propellants such as propane, butane, isobutene, dimethyl ether;
- styrene / PVP or styrene acrylamide copolymers;
- insect repellants such as butylacetylaminopropionate.

Preferred preservatives include

- phenoxyethanol;
- benzyl alcohol;
- methyl-, ethyl-, propyl-, butyl-, and isobutylparaben;
- zingerone.

Preferred perfumes are selected from the group consisting of limonene, citral, linalool, alpha-isomethylionon, geraniol, citronellol, 2-isobutyl-4-hydroxy-4-methyltetrahydropyrane, 2-tert.-pentylcyclohexylacetate, 3-methyl-5-phenyl-1-pentanol, 7-acetyl-1,1,3,4,4,6-hexamethyltetraline, adipine acid diester, alpha-amylcinnamaldehyde, alpha-methylionon, amyl C butylphenylmethylpropionalcinnamal, amylsalicylate, amylcinnamylalcohol, anisalcohol, benzoin, benzylalcohol, benzylbenzoate, benzylcinnamate, benzylsalicylate, bergamot oil, bitter orange oil, butylphenylmethylpropioal, cardamom oil, cedrol, cinnamal, cinnamylalcohol, citronnellylmethylcrotonate, lemon oil, coumarin, diethylsuccinate, ethyllinalool, eugenol, evernia furfuracea extracte, evernia prunastri extracte, farensol, guajak wood oil, hexylcinnamal, hexylsalicylate, hydroxycitronellal, lavender oil, lemon oil, linaylacetate, mandarine oil, menthyl PCA, methylheptenone, nutmeg oil, rosemary oil, sweet orange oil, terpineol, tonka bean oil, triethylcitrate, vanillin and combinations thereof.

In connection with the above preferred embodiments, it is to be understood that if the sunscreen or body-care composition comprises two or more additives, combinations of the additives as defined above are also part of the invention.

In connection with the above preferred embodiments, it is to be understood that the body-care product may further comprise water. In case water is present in the body-case product, the body-care product can be an oil in water emulsion (O/W emulsion) or a water in oil emulsion (W/O emulsion). According to a preferred embodiment of the present invention, the cosmetic or pharmaceutical composition comprising a compound of formula (I) is an O/W emulsion or a W/O emulsion.

The present invention is further illustrated by the following examples.

Examples

The following abbreviations are used herein:

| Abbreviation | Meaning |
|---|---|
| $D_2O$ | Deuterium oxide |
| g | gram(s) |
| MHz | megahertz |
| ml | milliliter |
| NMR | nuclear magnetic resonance |

The compounds of the present invention can be prepared according to the procedures of the following Schemes and Examples, using appropriate materials and are further exemplified by the following specific examples.

Unless otherwise specified, all starting materials are obtained from commercial suppliers and used without further purifications. Unless otherwise specified, all temperatures are expressed in °C and all reactions are conducted at rt.

Materials

Croconic acid was purchased from abcr GmbH, Germany, purity >95% [CAS Registry Number 488-86-8, 4,5-Dihydroxy-4-cyclopentene-1,2,3-trione].

Tinogard Q was purchased from BASF, Germany. Tinogard Q comprises tris(tetramethylhydroxypiperidinol) citrate in water and ethanol. Tinogard Q is an Excited State Quencher and a water soluble, yellowish-brown liquid.

Example 1: Syntheses of the compounds

Comparative Compound 1 (Comp. Compound 1): 4,5-Dihydroxy-4-cyclopentene-1,2,3-trione di-sodium salt

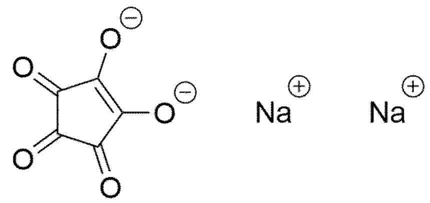

To a cooled solution of croconic acid (2.02g, 14.22 mmol) in water (30 ml), a 10% solution of sodium hydroxide in water (11.74 g, 29 mmol) was added slowly at 1 °C until the pH remained stable at 7±1. The resulting sodium salt precipitated partially as yellow crystals. The suspension was transferred to a larger round bottomed flask, 2-butanone (500 ml) was added and water/2-butanone was removed by azeotropic distillation. The resulting yellow crystals were suspended in a few milliliters of 2-butanone, filtered off, washed and dried in vacuo. The di-sodium salt (3.06 g) was obtained as yellow solid.

NMR ($^{13}C$, 100 MHz, $D_2O$): 188.4 ppm (only one single signal indicating the aromaticity of croconate di-anion).

Comparative Compound 2 (Comp. Compound 2): 4,5-Dihydroxy-4-cyclopentene-1,2,3-trione di-potassium salt

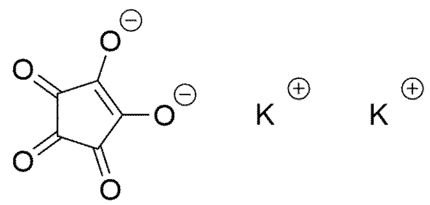

The di-potassium salt was achieved according to the Comparative Compound 1, whereas potassium hydroxide was used instead of sodium hydroxide.

NMR ($^{13}C$, 100 MHz, $D_2O$): 188.4 ppm (only one single signal indicating the aromaticity of croconate di-anion).

Compound A: 4,5-Dihydroxy-4-cyclopentene-1,2,3-trione di-triethanolammonium salt (herein also referred to as TEA croconic acid salt)

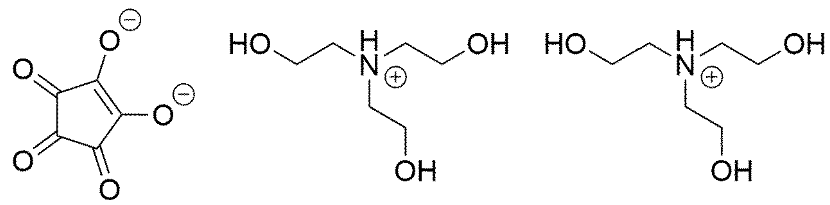

To a cooled solution of croconic acid (2.02g, 14.22 mmol) in water (30 ml) a 25% solution of triethanolamine in water (18.2g, 30.5 mmol) was added dropwise at 1 °C until the pH remained stable at 7±1. The ice bath was removed, and the reaction was warmed to ambient temperature. The suspension was transferred to a larger round bottomed flask, 2-butanone (500 ml) was added and water/2-butanone was removed by azeotropic distillation. The resulting triethanolammonium salt (7.0 g) was obtained as brown oil (highly hygroscopic).

NMR ($^{13}C$, 100 MHz, $D_2O$): 55.2, 55.3, 188.3 ppm (188.3 ppm signal indicating the aromaticity of croconate di-anion).

Example 2: Specific Extinction Coefficient

The specific extinction coefficient of Comparative (also referred to as Comp.) Compounds 1 and 2 and Compound A was measured using a Perkin Elmer Lambda 650 UV/VIS Spectrophotometer.

A stock solution of concentration c = 1mM of the UV-absorber was prepared in a 100ml volumetric flask using water. Dilution steps followed in order to adjust the concentration, such that the extinction E at optical pathlength d = 1 cm will be 1.0 ± 0.5. UV spectrum was determined in a cuvette of an optical pathlength of d = 1cm at 250nm to 500nm. With the Beer-Lambert law one obtains the molar decadic extinction coefficient at any wavelength with:

$$\varepsilon(\lambda) = \frac{E(\lambda)}{c \cdot d} \quad [\mathrm{l/(mol \cdot cm)}].$$

The respective specific extinction E%1,1cm can be obtained via the following equation:

$$E_{1,1}(\lambda) = \varepsilon(\lambda)[l/(mol \cdot cm)] \cdot \frac{10[g/l]}{M[g/mol]} \cdot 1[cm],$$

where M = molecular weight of the UV-absorber.

As can be derived from Table 1, all tested compounds are absorbing in an UV region relevant for skin protection. Hence, all tested compounds may be applicable for a cosmetic sun care per se.

Table 1. Maximum absorbance wavelength ($\lambda_{max}$) and specific extinction coefficient at $\lambda_{max}$ of Compound A and Comparative Compounds 1 and 2.

| Tested Compounds | $\lambda_{max}$ | E1,1 |
|---|---|---|
| Compound A | 364nm | 844 |
| Comp. Compound 1 | 364nm | 1996 |
| Comp. Compound 2 | 364nm | 1704 |

Example 3: Biodegradation

In general, the biodegradability is tested on the following concept:

$$\text{Test substance (DOC)} + O_2 \xrightarrow{\text{Biodegradation}} CO_2 + H_2O + \text{Biomass}$$

wherein DOC denotes Dissolved organic carbon.

The biodegradation of croconic acid, Myritol 318, Cetiol AB, and glucose was investigated according to OECD 301 (OECD GUIDELINE FOR TESTING OF CHEMICALS No 301) via the OECD 301F manometric respirometry test.

Figure 1A depicts the different degradation curves of readily biodegradable, slowly biodegradable, moderately biodegradable, and non-biodegradable. As can be seen from Figure 1B, croconic acid, Myritol 318, and Cetiol AB are readily biodegradable (glucose is a biodegradable reference).

When croconic acid is dissolved in water it dissociates into the di-anion and oxonium ions. Since the di-anion of croconic acid is responsible for UV absorption, only the biodegradation of croconic acid had been tested. It is however also known that 2,2',2"-nitrilotriethanol is readily biodegradable in water (see e.g. https://www.echa.europa.eu/web/guest/substance-information/-/substanceinfo/100.002.773 and https://www.echa.europa.eu/web/guest/registration-dossier/-/registered-dossier/15134/5/3/2). Thus, Compound A is biodegradable.

Example 4: Preparation of the Formulation

The Formulations 1 to 5 were prepared according to the following manufacturing process with the ingredients as disclosed in Tables 2 and 3.

Manufacturing process:

Part A was heated to 85°C. Part B was heated to 85°C. Part B was added into Part A under homogenization. The mixture was let cooled down to 30°C under moderate stirring and Part C was added. The mixture was cooled down under moderate stirring to room temperature.

Table 2. Formulation of cosmetic compositions (Formulations 1 to 3). INCI: International Nomenclature of Cosmetic Ingredients.

| | | | Formulation | | |
|---|---|---|---|---|---|
| | Trade name | INCI or chemical description | 1 | 2 | 3 |
| | | | w/w% | | |
| Part A | Emulgade PL68/50 | CetearylGlucoside (and) CetearylAlcohol | 1.5 | 1.5 | 1.5 |
| | Cutina GMS SE | Glyceryl stearate SE | 3 | 3 | 3 |
| | Lanette E | Sodium Cetearyl Sulfate | 1.5 | 1.5 | 1.5 |
| | Myritol 318 | Caprylic/Capric Triglyceride | 5 | 5 | 2.5 |
| | Cetiol AB | C12-15 alkyl benzoate | 10 | 10 | 4.5 |
| | Uvinul MC80 | Ethylhexyl methoxycinnamate (EHMC) | - | - | 8 |
| Part B | Water | Aqua | 68.1 | 63.5 | 67.1 |
| | Glycerine | 1-2 Propane diol | 2 | 2 | 2 |
| | Rheocare XGN | Xanthan Gum | 0.3 | 0.3 | 0.3 |
| | Comp. Compound 1 | 4,5-Dihydroxy-4-cyclopentene-1,2,3-trione di-sodium salt | 2.6 | - | - |
| | Compound A | 4,5-Dihydroxy-4-cyclopentene-1,2,3-trione di-triethanolammonium salt | - | 7.2 | 3.6 |
| Part C | Cetiol Ultimate | Undecane (and) Tridecane | 5 | 5 | 5 |
| | Sensiva SC10 | CaprylylGlycol (and) Ethylhexylglycerin | 1 | 1 | 1 |
| | Total | | 100 | 100 | 100 |

Table 3. Formulation of cosmetic compositions (Formulations 4 and 5). INCI: International Nomenclature of Cosmetic Ingredients.

| | | | Formulation | |
|---|---|---|---|---|
| | Trade name | INCI | 4 | 5 |
| | | | w/w% | |
| Part A | Eumulgin VL 75 | Lauryl Glucoside, Polyglyceryl-2 Dipolyhydroxystearate, Glycerin | 4 | 4 |
| | Cetiol B | Dibutyl Adipate | 8 | 8 |
| | Cetiol AB | C12-15 alkyl benzoate | 8 | 8 |
| | Myritol 331 | Cocoglycerides | 12 | 12 |
| | Lanette E | Sodium Cetearyl Sulfate | 1 | 1 |
| | Lanette O | CetearylAlcohol | 2 | 2 |
| Part B | Water | Aqua | 53.5 | 50.50 |
| | Glycerine | 1-2 Propane diol | 3 | 3 |
| | Rheocare XGN | Xanthan Gum | 0.3 | 0.3 |
| | Tinogard Q | Tris(Tetramethylhydroxypiperidinol) Citrate, Water and Ethanol | - | 3 |
| | Compound A | 4,5-Dihydroxy-4-cyclopentene-1,2,3-trione di-triethanolammonium salt | 7.2 | 7.2 |

(continued)

| | | | Formulation | |
|---|---|---|---|---|
| Part C | Protectol PE | Phenoxyethanol | 1 | 1 |
| | Total | | 100 | 100 |

Example 5. Absorbance of croconic acid salts in formulation

The absorbance of Formulation 1 and 2 was determined as follows:
30 mg milligram of product were evenly spread on roughened PMMA plates (Heliosplate SB6, Helioscreen Creil/France) using a presaturated finger cot. After an equilibration time of 15 minutes transmission measurements between 290 and 400 nm were carried out using a spectrophotometer equipped with an integrating sphere (UV Transmittance Analyzer UV1000-S, Labsphere North Sutton / US).

As can be seen from Figure 2, the absorbance of the formulation comprising Compound A is within the expected range, whereas the absorbance of the formulation comprising Comparative Compound 1 is lower (compare to Table 1), which is due to the crystallization of the compound after water evaporation.

Example 6. Photostability

Photostability of Formulations 3 to 5 was tested.

30 mg milligram of product were evenly spread on roughened PMMA plates (Heliosplate SB6, Helioscreen Creil/France) using a presaturated finger cot. After an equilibration time of 15 min. transmission measurements between 290 and 400 nm were carried out using a spectrophotometer equipped with an integrating sphere (UV Transmittance Analyzer UV1000-S, Labsphere North Sutton / US).

Afterwards various irradiation doses were applied on the plates using an Atlas CPS+ solar simulator (Atlas Material Testing, Linsengericht/Germany) equipped with a Xenon Arc lamp and special glass filter (Solar Standard 56077759).

Transmission measurements were repeated after irradiation doses of 5, 10, and 20, MED.

The samples of Formulation 3 were irradiated at 5 and 10 MED.

The samples of Formulation 4 were irradiated at 5 MED.

The samples of Formulation 5 were irradiated at 5, 10 and 20 MED.

In this connection it is noted that MED is the Minimal Erythemal Dose and is defined as the threshold dose that may produce sunburn. The susceptibility of skin to damages by a given erythemal effective UV dose depends from the individual skin type and the varying status of skin protection acquired in the course of the year, e.g. by tanning. The MED varies depending from skin.

Weighting of the COLIPA (the European Cosmetics, Toiletries and Perfumeries Association) standard sun spectrum with the erythemal action spectrum leads to the erythemal irradiance of 0.217 $W.m^{-2}$. Assuming that 1MED is achieved after a UV dose of 250 $J.m^{-2}$, the time corresponding to 1MED under COLIPA standard sun conditions would be 19 min. Since the UV intensity of the ATLAS Suntest CPS+ was by a factor of 1.68 higher than the COLIPA standard sun, the UV irradiance of the device may also be expressed as approximately 5 $MED.h^{-1}$.

As can be seen from Figure 3, Formulation 3 comprising Compound A as UVA filter and EHMC as UVB filter shows photoinstability in the UVA range.

Figure 4 demonstrates that Formulation 4 comprising Compound A as single UV filter is also not photostable.

In contrast thereto, Formulation 5 comprising Compound A as single UV filter in combination with the photostabilizer Tinogard Q showed an improved photostability.

## Claims

1. A compound of formula (I)

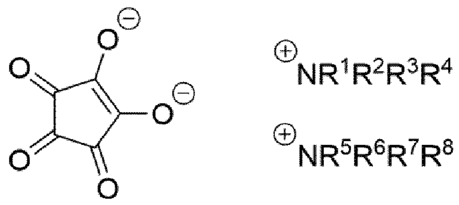

(I)

or a stereoisomer or tautomer thereof,
wherein
$R^1$ is H or $C_1$-$C_4$-alkyl;
$R^2$ is H, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH;
$R^3$ and $R^4$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; or together with the nitrogen to which they are bonded form a 5-membered saturated, partially or fully unsaturated, or aromatic heterocyclic ring, wherein said heterocyclic ring comprises one or more, same or different heteroatoms selected from O, N, or S, wherein said N- and/or S-atoms are independently oxidized or non-oxidized, and wherein each substitutable carbon or heteroatom in the heterocyclic ring is independently unsubstituted or substituted with one or more, same or different substituents $R^M$; or together with the nitrogen to which they are bonded form a double bond to a carbon atom, which is further substituted with two N-atoms, which are independently unsubstituted or substituted with one or more, same or different substituents $R^N$;
$R^5$ is H or $C_1$-$C_4$-alkyl;
$R^6$ is H, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH;
$R^7$ and $R^8$ are independently $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH; or together with the nitrogen to which they are bonded form a 5-membered saturated, partially or fully unsaturated, or aromatic heterocyclic ring, wherein said heterocyclic ring comprises one or more, same or different heteroatoms selected from O, N, or S, wherein said N- and/or S-atoms are independently oxidized or non-oxidized, and wherein each substitutable carbon or heteroatom in the heterocyclic ring is independently unsubstituted or substituted with one or more, same or different substituents $R^M$; or together with the nitrogen to which they are bonded form a double bond to a carbon atom, which is further substituted with two N-atoms, which are independently unsubstituted or substituted with one or more, same or different substituents $R^N$;
$R^M$ is halogen, CN, $NO_2$, $NH_2$, OH, $C_1$-$C_2$-alkyl, $C_1$-$C_2$-alkoxy, C(=O)$R^X$, or two $R^M$ form =O;
$R^N$ is $C_1$-$C_8$-alkyl, $C_1$-$C_6$-hydroxyalkyl, $C_2$-$C_4$-hydroxyalkenyl, $C_1$-$C_6$-aminoalkyl, $(CH_2O)_n$-OH, $(CH_2CH_2O)_n$-OH, or $(CH_2CH_2CH_2O)_n$-OH, wherein each substitutable carbon atom is independently unsubstituted or substituted with one or more, same or different substituents $R^Y$;
$R^X$ is H, $C_1$-$C_2$-alkyl, phenyl, or benzyl;
$R^Y$ is halogen, CN, $NO_2$, $NH_2$, OH, C(=O)$R^X$, or two $R^Y$ form =O; and
n is an integer from 1 to 10.

**2.** The compound according to claim 1, wherein
$R^1$ and $R^5$ are H.

**3.** The compound according to claim 1 or 2, wherein

$R^2$ to $R^4$ are independently $C_1$-$C_6$-hydroxyalkyl; and
$R^6$ to $R^8$ are independently $C_1$-$C_6$-hydroxyalkyl.

**4.** The compound according to any one of claims 1 to 3, wherein

$R^1$ and $R^5$ are the same, preferably H;
$R^2$ to $R^4$ are the same; and
$R^6$ to $R^8$ are the same.

**5.** The compound according to any one of claims 1 to 4, wherein
$R^2$ to $R^4$ and $R^6$ to $R^8$ are hydroxyethyl.

**6.** The compound according to claim 1 or 2, wherein

$R^1$, $R^2$, $R^5$, and $R^6$are the same;
$R^3$ and $R^4$ together with the nitrogen to which they are bonded form a double bond to a carbon atom, which is further substituted with two N-atoms, which are independently unsubstituted or substituted with one or more, same or different substituents $R^N$;
$R^7$ and $R^8$ together with the nitrogen to which they are bonded form a double bond to a carbon atom, which is further substituted with two N-atoms, which are independently unsubstituted or substituted with one or more, same or different substituents $R^N$;
$R^N$ is $C_1$-$C_6$-alkyl, wherein each substitutable carbon atom is independently unsubstituted or substituted with one or more, same or different substituents $R^Y$; and
$R^Y$ is $NH_2$, OH, or two $R^Y$ form =O.

**7.** The compound according to any one of claims 1, 2 or 6, wherein

$R^1$, $R^2$, $R^5$, and $R^6$ are H;
$R^3$ and $R^4$ together with the nitrogen to which they are bonded form a double bond to a carbon atom, which is further substituted with two N-atoms, wherein one of these two N-atoms is substituted with $R^N$;
$R^7$ and $R^8$ together with the nitrogen to which they are bonded form a double bond to a carbon atom, which is further substituted with two N-atoms, wherein one of these two N-atoms is substituted with $R^N$;
$R^N$ is $C_2$-$C_5$-alkyl, wherein each substitutable carbon atom is independently unsubstituted or substituted with one or more, same or different substituents $R^Y$; and
$R^Y$ is $NH_2$, OH, or two $R^Y$ form =O.

**8.** The compound according to claim 1 or 2, wherein

$R^1$, $R^2$, $R^5$, and $R^6$ are H;
$R^3$ and $R^4$ together with the nitrogen to which they are bonded form a double bond to a carbon atom, which is further substituted with two $NH_2$; and
$R^7$ and $R^8$ together with the nitrogen to which they are bonded form a double bond to a carbon atom, which is further substituted with two $NH_2$.

**9.** The compound according to claim 1, wherein the compound according to formula (I) is selected from the group consisting of 4,5-dihydroxy-4-cyclopentene-1,2,3-trione di-triethanolammonium salt, 4,5-dihydroxy-4-cyclopentene-1,2,3-trione di-guanidinium salt, and 4,5-dihydroxy-4-cyclopentene-1,2,3-trione di-argininium salt.

**10.** A cosmetic or pharmaceutical composition, comprising a compound according to any one of claims 1 to 9 and optionally a dermatologically acceptable emulsifier, thickener, or emollient.

**11.** The cosmetic or pharmaceutical composition according to claim 10, further comprising at least one additional UV filter, different to the compound of formula (I), preferably wherein the at least one additional UV filter is a natural and/or biodegradable UV filter.

**12.** The cosmetic or pharmaceutical composition according to claim 10 and 11, further comprising a photostabilizer, preferably a quencher, wherein preferably the photostabilizer is comprised in the cosmetic or pharmaceutical composition in an amount of at least 0.5 wt.-%, based on the total weight of the cosmetic or pharmaceutical composition.

**13.** The at least one compound according to any one of claims 1 to 9 or the cosmetic or pharmaceutical composition according to claim 10, 11, or 12 for use to protect skin against UV radiations.

**14.** Use of a compound according to any one of claims 1 to 9 in body-care products or household cleaning and treating agents as a UV filter.

**Patentansprüche**

**1.** Verbindung der Formel (I)

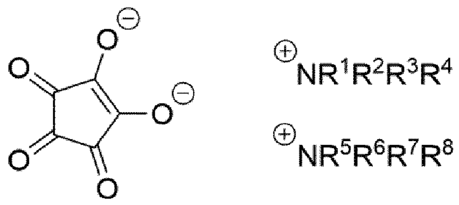

(I)

oder Stereoisomer oder Tautomer davon,
wobei
$R^1$ für H oder $C_1$-$C_4$-Alkyl steht;
$R^2$ für H, $C_1$-$C_6$-Hydroxyalkyl, $C_2$-$C_4$-Hydroxyalkenyl, $C_1$-$C_6$-Aminoalkyl, $(CH_2O)_n$-OH, $(CH_2CH_2O)_n$-OH oder $(CH_2CH_2CH_2O)_n$-OH steht;
$R^3$ und $R^4$ unabhängig für $C_1$-$C_6$-Hydroxyalkyl, $C_2$-$C_4$-Hydroxyalkenyl, $C_1$-$C_6$-Aminoalkyl, $(CH_2O)_n$-OH, $(CH_2CH_2O)_n$-OH oder $(CH_2CH_2CH_2O)_n$-OH stehen oder zusammen mit dem Stickstoff, an den sie gebunden sind, einen 5-gliedrigen gesättigten, teilweise oder vollständig ungesättigten oder aromatischen heterocyclischen Ring bilden, wobei der heterocyclische Ring ein oder mehrere, gleiche oder verschiedene Heteroatome, die aus O, N oder S ausgewählt sind, umfasst, wobei die N- und/oder S-Atome unabhängig oxidiert oder nicht oxidiert sind und wobei jedes substituierbare Kohlenstoff- oder Heteroatom in dem heterocyclischen Ring unabhängig unsubstituiert oder durch einen oder mehrere, gleiche oder verschiedene Substituenten $R^M$ substituiert ist; oder zusammen mit dem Stickstoff, an den sie gebunden sind, eine Doppelbindung zu einem Kohlenstoffatom bilden, welches weiter durch zwei N-Atome substituiert ist, welche unabhängig unsubstituiert oder durch einen oder mehrere, gleiche oder verschiedene Substituenten $R^N$ substituiert sind;
$R^5$ für H oder $C_1$-$C_4$-Alkyl steht;
$R^6$ für H, $C_1$-$C_6$-Hydroxyalkyl, $C_2$-$C_4$-Hydroxyalkenyl, $C_1$-$C_6$-Aminoalkyl, $(CH_2O)_n$-OH, $(CH_2CH_2O)_n$-OH oder $(CH_2CH_2CH_2O)_n$-OH steht;
$R^7$ und $R^8$ unabhängig für $C_1$-$C_6$-Hydroxyalkyl, $C_2$-$C_4$-Hydroxyalkenyl, $C_1$-$C_6$-Aminoalkyl, $(CH_2O)_n$-OH, $(CH_2CH_2O)_n$-OH oder $(CH_2CH_2CH_2O)_n$-OH stehen oder zusammen mit dem Stickstoff, an den sie gebunden sind, einen 5-gliedrigen gesättigten, teilweise oder vollständig ungesättigten oder aromatischen heterocyclischen Ring bilden, wobei der heterocyclische Ring ein oder mehrere, gleiche oder verschiedene Heteroatome, die aus O, N oder S ausgewählt sind, umfasst, wobei die N- und/oder S-Atome unabhängig oxidiert oder nicht oxidiert sind und wobei jedes substituierbare Kohlenstoff- oder Heteroatom in dem heterocyclischen Ring unabhängig unsubstituiert oder durch einen oder mehrere, gleiche oder verschiedene Substituenten $R^M$ substituiert ist; oder zusammen mit dem Stickstoff, an den sie gebunden sind, eine Doppelbindung zu einem Kohlenstoffatom bilden, welches weiter durch zwei N-Atome substituiert ist, welche unabhängig unsubstituiert oder durch einen oder mehrere, gleiche oder verschiedene Substituenten $R^N$ substituiert sind;
$R^M$ für Halogen, CN, $NO_2$, $NH_2$, OH, $C_1$-$C_2$-Alkyl, $C_1$-$C_2$-Alkoxy, $C(=O)R^X$ steht oder zwei $R^M$ =O bilden;
$R^N$ für $C_1$-$C_8$-Alkyl, $C_1$-$C_6$-Hydroxyalkyl, $C_2$-$C_4$-Hydroxyalkenyl, $C_1$-$C_6$-Aminoalkyl, $(CH_2O)_n$-OH, $(CH_2CH_2O)_n$-OH oder $(CH_2CH_2CH_2O)_n$-OH steht, wobei jedes substituierbare Kohlenstoffatom unabhängig unsubstituiert oder durch einen oder mehrere, gleiche oder verschiedene Substituenten $R^Y$ substituiert ist;
$R^X$ für H, $C_1$-$C_2$-Alkyl, Phenyl oder Benzyl steht;
$R^Y$ für Halogen, CN, $NO_2$, $NH_2$, OH, $C(=O)R^X$ steht oder zwei $R^Y$ =O bilden; und
n für eine ganze Zahl von 1 bis 10 steht.

**2.** Verbindung nach Anspruch 1, wobei
$R^1$ und $R^5$ für H stehen.

**3.** Verbindung nach Anspruch 1 oder 2, wobei

$R^2$ bis $R^4$ unabhängig für $C_1$-$C_6$-Hydroxyalkyl stehen; und
$R^6$ bis $R^8$ unabhängig für $C_1$-$C_6$-Hydroxyalkyl stehen.

**4.** Verbindung nach einem der Ansprüche 1 bis 3, wobei

$R^1$ und $R^5$ gleich sind und vorzugsweise für H stehen;
$R^2$ bis $R^4$ gleich sind; und
$R^6$ bis $R^8$ gleich sind.

**5.** Verbindung nach einem der Ansprüche 1 bis 4, wobei $R^2$ bis $R^4$ und $R^6$ bis $R^8$ für Hydroxyethyl stehen.

**6.** Verbindung nach Anspruch 1 oder 2, wobei

$R^1$, $R^2$, $R^5$ und $R^6$ gleich sind;
$R^3$ und $R^4$ zusammen mit dem Stickstoff, an den sie gebunden sind, eine Doppelbindung zu einem Kohlenstoffatom bilden, welches weiter durch zwei N-Atome substituiert ist, welche unabhängig unsubstituiert oder durch einen oder mehrere, gleiche oder verschiedene Substituenten $R^N$ substituiert sind;
$R^7$ und $R^8$ zusammen mit dem Stickstoff, an den sie gebunden sind, eine Doppelbindung zu einem Kohlenstoffatom bilden, welches weiter durch zwei N-Atome substituiert ist, welche unabhängig unsubstituiert oder durch einen oder mehrere, gleiche oder verschiedene Substituenten $R^N$ substituiert sind;
$R^N$ für $C_1$-$C_6$-Alkyl steht, wobei jedes substituierbare Kohlenstoffatom unabhängig unsubstituiert oder durch einen oder mehrere, gleiche oder verschiedene Substituenten $R^Y$ substituiert ist; und
$R^Y$ für $NH_2$, OH steht oder zwei $R^Y$ =O bilden.

**7.** Verbindung nach einem der Ansprüche 1, 2 oder 6, wobei

$R^1$, $R^2$, $R^5$ und $R^6$ für H stehen;
$R^3$ und $R^4$ zusammen mit dem Stickstoff, an den sie gebunden sind, eine Doppelbindung zu einem Kohlenstoffatom bilden, welches weiter durch zwei N-Atome substituiert ist, wobei eines dieser beiden N-Atome durch $R^N$ substituiert ist;
$R^7$ und $R^8$ zusammen mit dem Stickstoff, an den sie gebunden sind, eine Doppelbindung zu einem Kohlenstoffatom bilden, welches weiter durch zwei N-Atome substituiert ist, wobei eines dieser beiden N-Atome durch $R^N$ substituiert ist;
$R^N$ für $C_2$-$C_5$-Alkyl steht, wobei jedes substituierbare Kohlenstoffatom unabhängig unsubstituiert oder durch einen oder mehrere, gleiche oder verschiedene Substituenten $R^Y$ substituiert ist; und
$R^Y$ für $NH_2$, OH steht oder zwei $R^Y$ =O bilden.

**8.** Verbindung nach Anspruch 1 oder 2, wobei

$R^1$, $R^2$, $R^5$ und $R^6$ für H stehen;
$R^3$ und $R^4$ zusammen mit dem Stickstoff, an den sie gebunden sind, eine Doppelbindung zu einem Kohlenstoffatom bilden, welches weiter durch zwei $NH_2$ substituiert ist; und
$R^7$ und $R^8$ zusammen mit dem Stickstoff, an den sie gebunden sind, eine Doppelbindung an ein Kohlenstoffatom bilden, das weiter durch zwei $NH_2$ substituiert ist.

**9.** Verbindung nach Anspruch 1, wobei die Verbindung gemäß Formel (I) aus der Gruppe bestehend aus 4,5-Dihydroxy-4-cyclopenten-1,2,3-trion-Ditriethanolammoniumsalz, 4,5-Dihydroxy-4-cyclopenten-1,2,3-trion-Diguanidiniumsalz und 4,5-Dihydroxy-4-cyclopenten-1,2,3-trion-Diargininiumsalz ausgewählt ist.

**10.** Kosmetische oder pharmazeutische Zusammensetzung, umfassend eine Verbindung nach einem der Ansprüche 1 bis 9 und gegebenenfalls einen dermatologisch unbedenklichen Emulgator, einen dermatologisch unbedenklichen Verdicker oder ein dermatologisch unbedenkliches Emolliens.

**11.** Kosmetische oder pharmazeutische Zusammensetzung nach Anspruch 10, ferner umfassend mindestens einen zusätzlichen UV-Filter, der von der Verbindung der Formel (I) verschieden ist, vorzugsweise wobei es sich bei dem mindestens einen zusätzlichen UV-Filter um einen natürlichen und/oder biologisch abbaubaren UV-Filter handelt.

**12.** Kosmetische oder pharmazeutische Zusammensetzung nach Anspruch 10 und 11, ferner umfassend einen Photostabilisator, vorzugsweise einen Quencher, wobei der Photostabilisator vorzugsweise in der kosmetischen oder pharmazeutischen Zusammensetzung in einer Menge von mindestens 0,5 Gew.-%, bezogen auf das Gesamtgewicht der kosmetischen oder pharmazeutischen Zusammensetzung, enthalten ist.

**13.** Verbindung(en) nach einem der Ansprüche 1 bis 9 oder kosmetische oder pharmazeutische Zusammensetzung nach Anspruch 10, 11 oder 12 zur Verwendung zum Schutz der Haut vor UV-Strahlung.

**14.** Verwendung einer Verbindung nach einem der Ansprüche 1 bis 9 in Körperpflegemitteln oder Haushaltsreinigungs- und -behandlungsmitteln als UV-Filter.

## Revendications

**1.** Composé de formule (I)

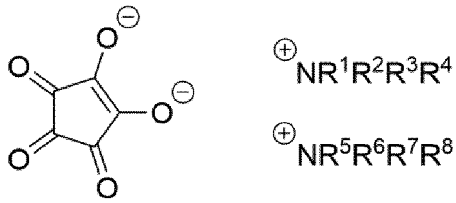

(I)

ou stéréoisomère ou tautomère de celui-ci,
dans lequel
$R^1$ est H ou alkyle en $C_1$-$C_4$ ;
$R^2$ est H, hydroxyalkyle en $C_1$-$C_6$, hydroxyalcényle en $C_2$-$C_4$, aminoalkyle en $C_1$-$C_6$, $(CH_2O)_n$-OH, $(CH_2CH_2O)_n$-OH ou $(CH_2CH_2CH_2O)_n$-OH ;
$R^3$ et $R^4$ sont indépendamment hydroxyalkyle en $C_1C_6$, hydroxyalcényle en $C_2$-$C_4$, aminoalkyle en $C_1$-$C_6$, $(CH_2O)_n$-OH, $(CH_2CH_2O)_n$-OH ou $(CH_2CH_2CH_2O)_n$-OH ; ou forment conjointement avec l'azote auquel ils sont liés un cycle hétérocyclique à 5 chaînons saturé, partiellement ou totalement insaturé ou aromatique, ledit cycle hétérocyclique comprenant un ou plusieurs hétéroatomes identiques ou différents choisis parmi O, N ou S, lesdits atomes de N et/ou S étant indépendamment oxydés ou non oxydés et chaque carbone ou hétéroatome pouvant être substitué présent dans le cycle hétérocyclique étant indépendamment non substitué ou substitué par un ou plusieurs substituants $R^M$ identiques ou différents ; ou forment conjointement avec l'azote auquel ils sont liés une double liaison à un atome de carbone, qui est en outre substitué par deux atomes de N, qui sont indépendamment non substitués ou substitués par un ou plusieurs substituants $R^N$ identiques ou différent ;
$R^5$ est H ou alkyle en $C_1$-$C_4$ ;
$R^6$ est H, hydroxyalkyle en $C_1$-$C_6$, hydroxyalcényle en $C_2$-$C_4$, aminoalkyle en $C_1$-$C_6$, $(CH_2O)_n$-OH, $(CH_2CH_2O)_n$-OH ou $(CH_2CH_2CH_2O)_n$-OH ;
$R^7$ et $R^8$ sont indépendamment hydroxyalkyle en $C_1$-$C_6$, hydroxyalcényle en $C_2$-$C_4$, aminoalkyle en $C_1$-$C_6$, $(CH_2O)_n$-OH, $(CH_2CH_2O)_n$-OH ou $(CH_2CH_2CH_2O)_n$-OH ; ou forment conjointement avec l'azote auquel ils sont liés un cycle hétérocyclique à 5 chaînons saturé, partiellement ou totalement insaturé ou aromatique, ledit cycle hétérocyclique comprenant un ou plusieurs hétéroatomes identiques ou différents choisis parmi O, N ou S, lesdits atomes de N et/ou S étant indépendamment oxydés ou non oxydés et chaque carbone ou hétéroatome pouvant être substitué présent dans le cycle hétérocyclique étant indépendamment non substitué ou substitué par un ou plusieurs substituants $R^M$ identiques ou différents ; ou forment conjointement avec l'azote auquel ils sont liés une double liaison à un atome de carbone, qui est en outre substitué par deux atomes de N, qui sont indépendamment non substitués ou substitués par un ou plusieurs substituants $R^N$ identiques ou différent ;
$R^M$ est halogène, CN, $NO_2$, $NH_2$, OH, alkyle en $C_1$-$C_2$, alcoxy en $C_1$-$C_2$, C(=O)$R^X$ ou deux $R^M$ forment =O ;
$R^N$ est alkyle en $C_1$-$C_8$, hydroxyalkyle en $C_1$-$C_6$, hydroxyalcényle en $C_2$-$C_4$, aminoalkyle en $C_1$-$C_6$, $(CH_2O)_n$-OH, $(CH_2CH_2O)_n$-OH ou $(CH_2CH_2CH_2O)_n$-OH, chaque atome de carbone pouvant être substitué étant indépendamment non substitué ou substitué par un ou plusieurs substituants $R^Y$ identiques ou différents ;
$R^X$ est H, alkyle en $C_1$-$C_2$, phényle ou benzyle ;
$R^Y$ est halogène, CN, $NO_2$, $NH_2$, OH, C(=O)$R^X$ ou deux $R^Y$ forment =O ; et
n est un nombre entier allant de 1 à 10.

**2.** Composé selon la revendication 1, dans lequel
$R^1$ et $R^5$ sont H.

**3.** Composé selon la revendication 1 ou 2, dans lequel

$R^2$ à $R^4$ sont indépendamment hydroxyalkyle en $C_1$-$C_6$ ; et
$R^6$ à $R^8$ sont indépendamment hydroxyalkyle en $C_1$-$C_6$.

**4.** Composé selon l'une quelconque des revendications 1 à 3, dans lequel

$R^1$ et $R^5$ sont identiques, de préférence H ;
$R^2$ à $R^4$ sont identiques ; et
$R^6$ à $R^8$ sont identiques.

**5.** Composé selon l'une quelconque des revendications 1 à 4, dans lequel
$R^2$ à $R^4$ et $R^6$ à $R^8$ sont hydroxyéthyle.

**6.** Composé selon la revendication 1 ou 2, dans lequel

$R^1$, $R^2$, $R^5$ et $R^6$ sont identiques ;
$R^3$ et $R^4$ forment conjointement avec l'azote auquel ils sont liés une double liaison à un atome de carbone, qui est en outre substitué par deux atomes de N, qui sont indépendamment non substitués ou substitués par un ou plusieurs substituants $R^N$ identiques ou différents ;
$R^7$ et $R^8$ conjointement avec l'azote auquel ils sont liés forment une double liaison à un atome de carbone, qui est en outre substitué par deux atomes de N, qui sont indépendamment non substitués ou substitués par un ou plusieurs substituants $R^N$ identiques ou différents ;
$R^N$ est alkyle en $C_1$-$C_6$, chaque atome de carbone pouvant être substitué étant indépendamment non substitué ou substitué par un ou plusieurs substituants $R^Y$ identiques ou différents ; et
$R^Y$ est $NH_2$, OH ou deux $R^Y$ forment =O.

**7.** Composé selon l'une quelconque des revendications 1, 2 ou 6, dans lequel

$R^1$, $R^2$, $R^5$ et $R^6$ sont H ;
$R^3$ et $R^4$ forment conjointement avec l'azote auquel ils sont liés une double liaison à un atome de carbone, qui est en outre substitué par deux atomes de N, l'un de ces deux atomes de N étant substitué par $R^N$ ;
$R^7$ et $R^8$ forment conjointement avec l'azote auquel ils sont liés une double liaison à un atome de carbone, qui est en outre substitué par deux atomes de N, l'un de ces deux atomes de N étant substitué par $R^N$ ;
$R^N$ est alkyle en $C_2$-$C_5$, chaque atome de carbone pouvant être substitué étant indépendamment non substitué ou substitué par un ou plusieurs substituants $R^Y$ identiques ou différents ; et
$R^Y$ est $NH_2$, OH ou deux $R^Y$ forment =O.

**8.** Composé selon la revendication 1 ou 2, dans lequel

$R^1$, $R^2$, $R^5$ et $R^6$ sont H ;
$R^3$ et $R^4$ forment conjointement avec l'azote auquel ils sont liés une double liaison à un atome de carbone, qui est en outre substitué par deux $NH_2$ ; et
$R^7$ et $R^8$ forment conjointement avec l'azote auquel ils sont liés une double liaison à un atome de carbone, qui est en outre substitué par deux $NH_2$.

**9.** Composé selon la revendication 1, le composé selon la formule (I) étant choisi dans le groupe constitué par le sel de ditriéthanolammonium de la 4,5-dihydroxy-4-cyclopentène-1,2,3-trione, le sel de diguanidinium de la 4,5-dihydroxy-4-cyclopentène-1,2,3-trione et le sel de diargininium de la 4,5-dihydroxy-4-cyclopentène-1,2,3-trione.

**10.** Composition cosmétique ou pharmaceutique, comprenant un composé selon l'une quelconque des revendications 1 à 9 et éventuellement un émulsifiant, épaississant ou émollient dermatologiquement acceptable.

**11.** Composition cosmétique ou pharmaceutique selon la revendication 10, comprenant en outre au moins un filtre UV supplémentaire, différent du composé de formule (I), de préférence l'au moins un filtre UV supplémentaire étant un filtre UV naturel et/ou biodégradable.

**12.** Composition cosmétique ou pharmaceutique selon les revendications 10 et 11, comprenant en outre un photostabilisant, de préférence un agent photostabilisant par extinction, de préférence le photostabilisant étant compris dans la composition cosmétique ou pharmaceutique en une quantité d'au moins 0,5 % en poids, par rapport au poids total de la composition cosmétique ou pharmaceutique.

**13.** Composé selon l'une quelconque des revendications 1 à 9 ou composition cosmétique ou pharmaceutique selon la

revendication 10, 11 ou 12, destinés à être utilisés pour protéger la peau des rayonnements UV.

**14.** Utilisation d'un composé selon l'une quelconque des revendications 1 à 9 dans des produits de soins corporels ou des agents de nettoyage et de traitement ménagers en tant que filtre UV.

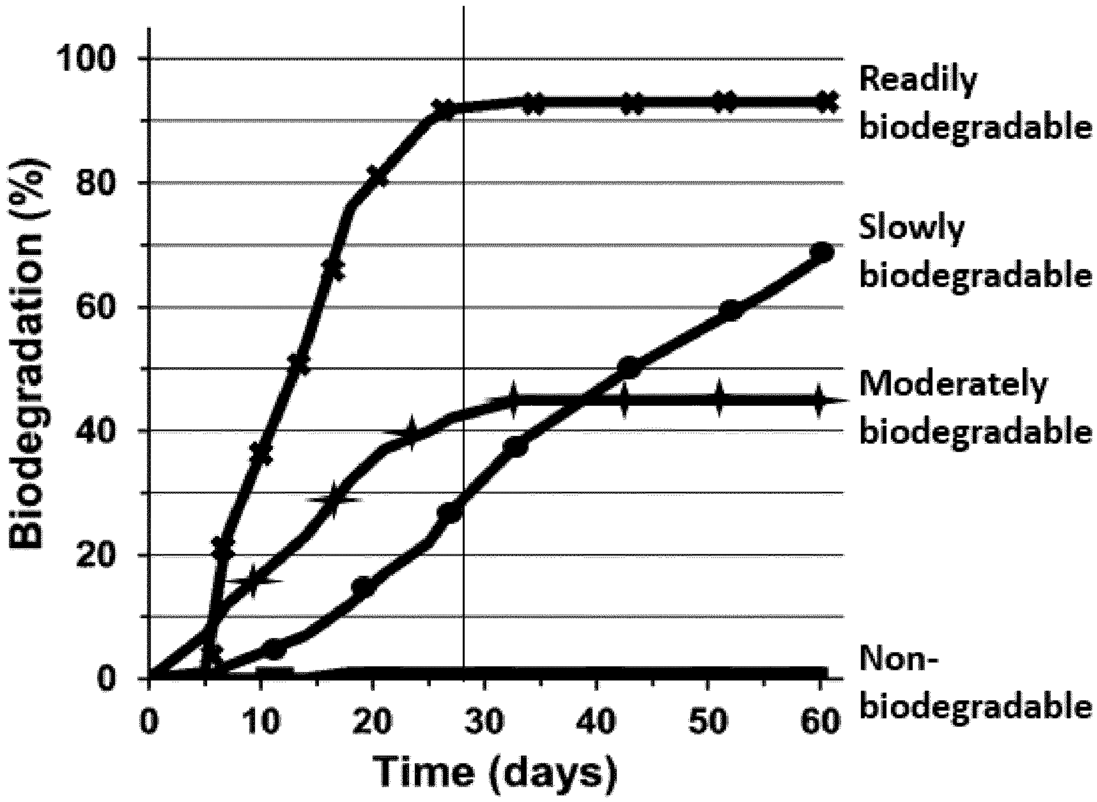
100
80
60
40
20
0
Biodegradation (%)
0
10
20
30
40
50
60
Time (days)
Readily biodegradable
Slowly biodegradable
Moderately biodegradable
Non-biodegradable

Fig. 1A

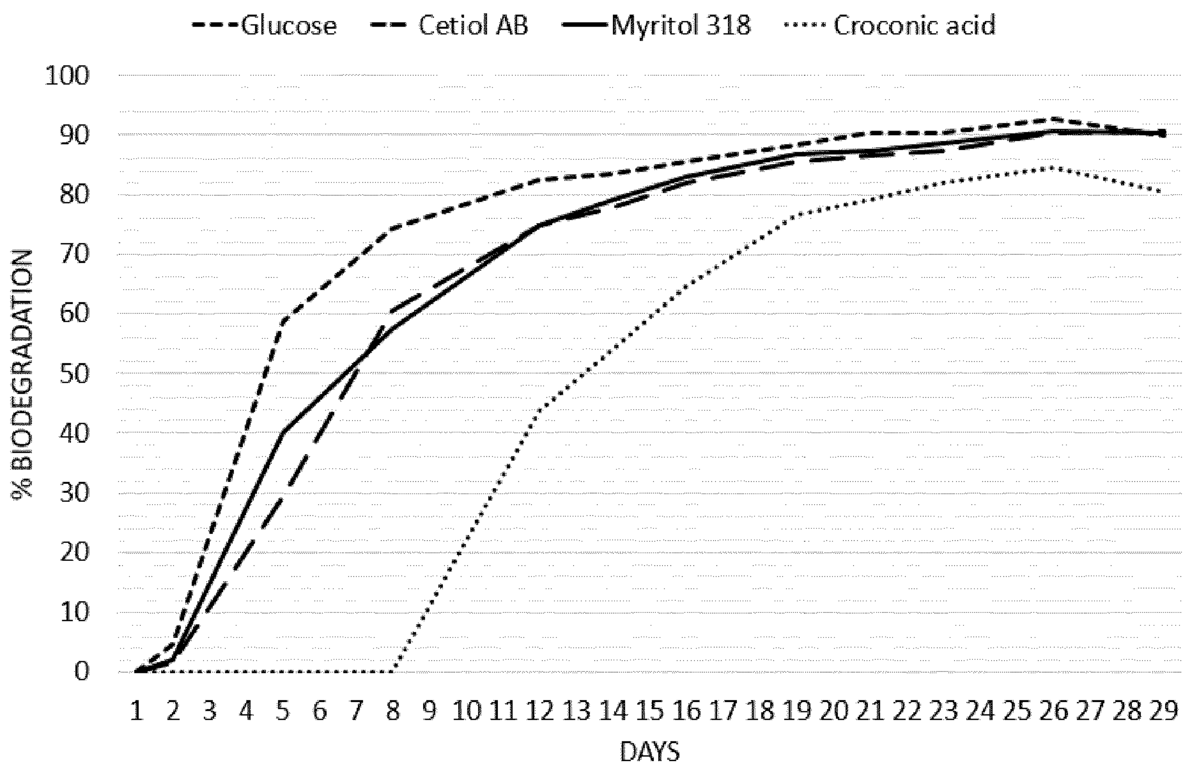
Glucose
Cetiol AB
Myritol 318
Croconic acid
100
90
80
70
60
50
40
30
20
10
0
% BIODEGRADATION
1 2 3 4 5 6 7 8 9 10 11 12 13 14 15 16 17 18 19 20 21 22 23 24 25 26 27 28 29
DAYS

Fig. 1B

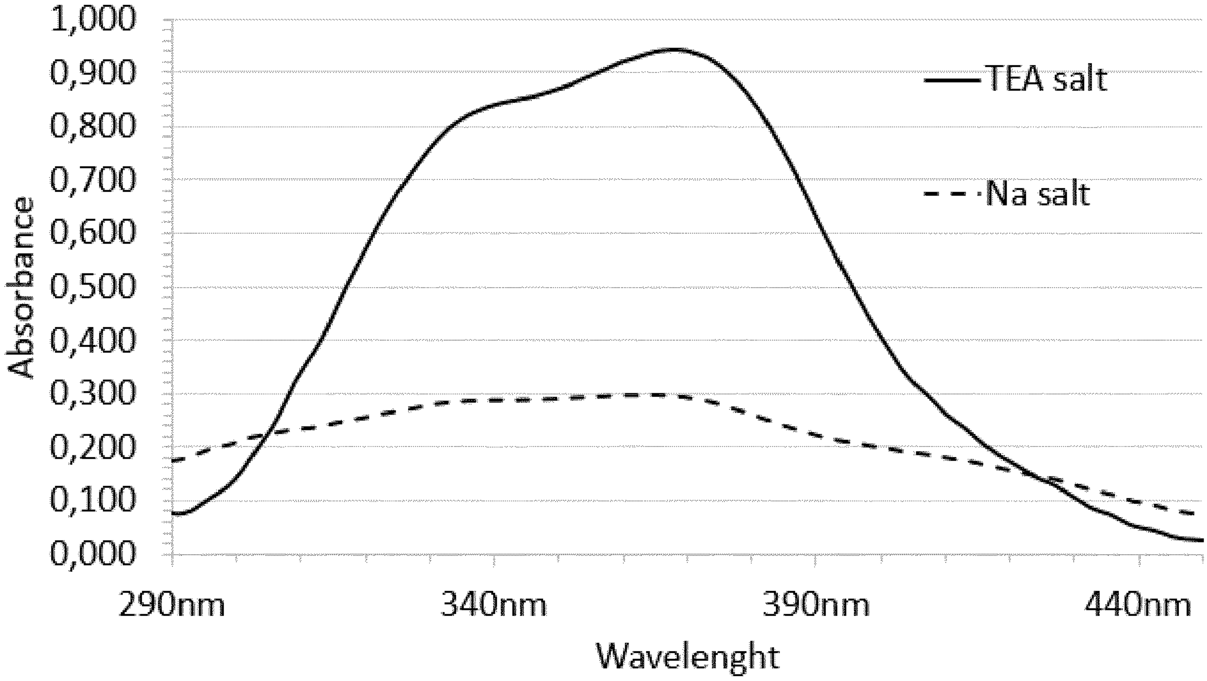
1,000
0,900
0,800
0,700
0,600
0,500
0,400
0,300
0,200
0,100
0,000
Absorbance
TEA salt
Na salt
290nm
340nm
390nm
440nm
Wavelenght

Fig. 2

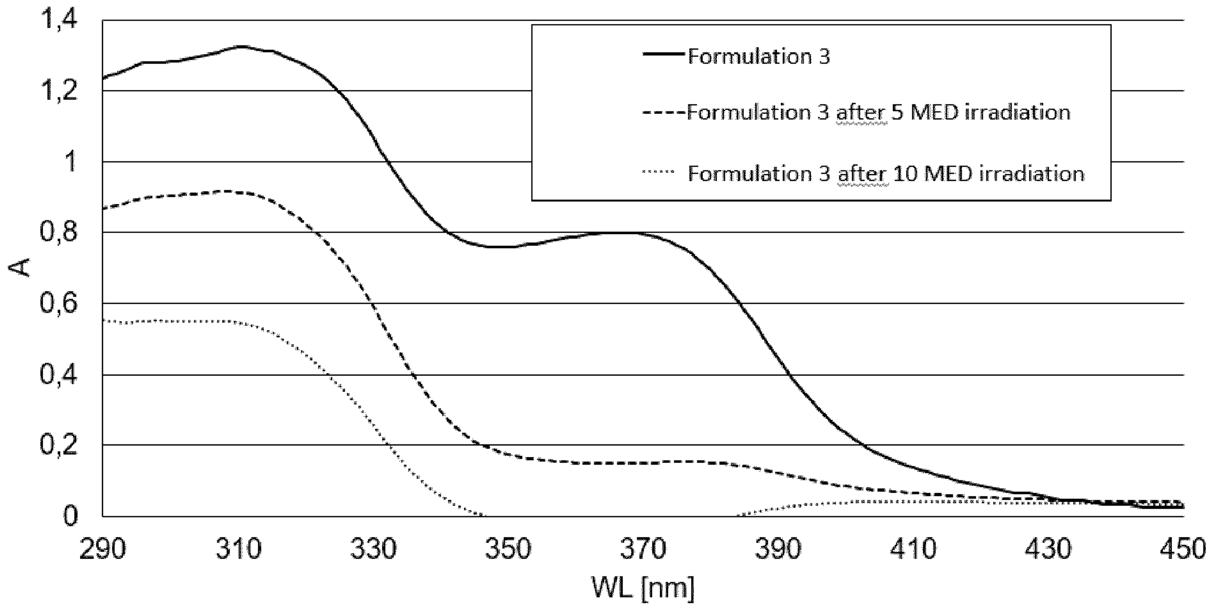
1,4
1,2
1
0,8
0,6
0,4
0,2
0
A
Formulation 3
Formulation 3 after 5 MED irradiation
Formulation 3 after 10 MED irradiation
290
310
330
350
370
390
410
430
450
WL [nm]

Fig. 3

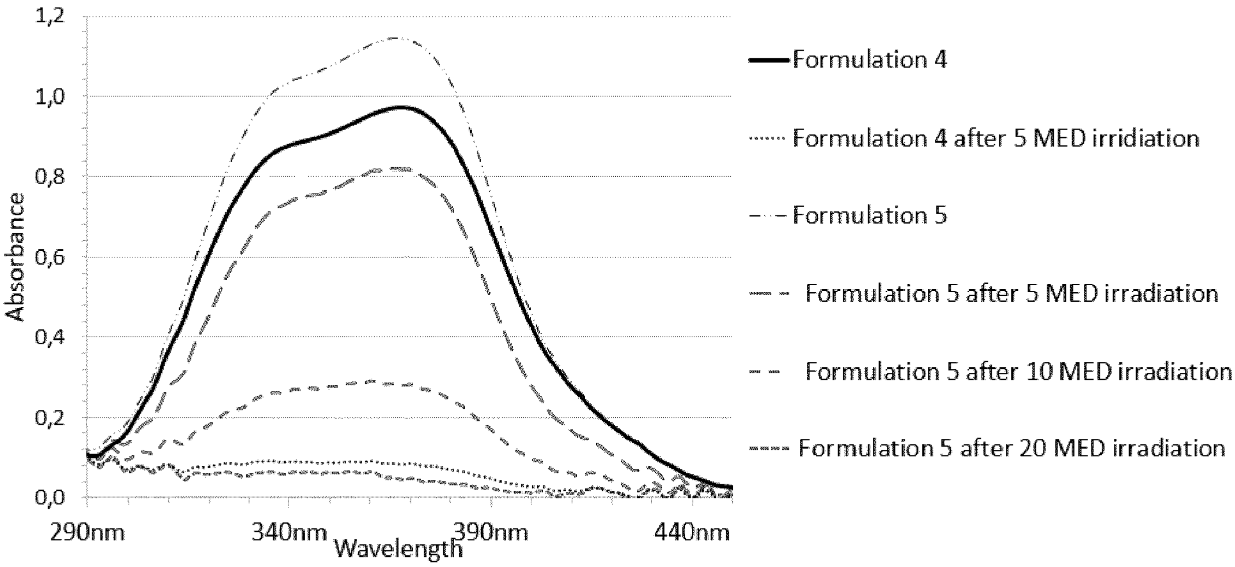
1,2
1,0
0,8
0,6
0,4
0,2
0,0
Absorbance
290nm
340nm
390nm
440nm
Wavelength
Formulation 4
Formulation 4 after 5 MED irridiation
Formulation 5
Formulation 5 after 5 MED irradiation
Formulation 5 after 10 MED irradiation
Formulation 5 after 20 MED irradiation

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FABER et al.** *Electrochimica Acta*, 2000, vol. 45 (17), 2697-2705
- **BAKI et al.** Introduction to Cosmetic Formulation and Technology. John Wiley & Sons, 2015, 244
- **FABRE et al.** *Can. J. Chem.*, 1995, vol. 73, 1298-1304
- **MAUER et al.** *Pharmaceutical Development and Technology*, 2010, vol. 15 (6), 582-594
- *CHEMICAL ABSTRACTS*, 444811-29-4
- *CHEMICAL ABSTRACTS*, 477844-93-2
- *CHEMICAL ABSTRACTS*, 127474-91-3
- *CHEMICAL ABSTRACTS*, 68890-66-4
- *CHEMICAL ABSTRACTS*, 1750-49-8
- *CHEMICAL ABSTRACTS*, 2078-71-9
- *CHEMICAL ABSTRACTS*, 872424-70-9
- *CHEMICAL ABSTRACTS*, 872424-71-0
- *CHEMICAL ABSTRACTS*, 872424-72-1
- *CHEMICAL ABSTRACTS*, 872424-73-2
- *CHEMICAL ABSTRACTS*, 6197-30-4
- *CHEMICAL ABSTRACTS*, 118-60-5
- *CHEMICAL ABSTRACTS*, 180898-37-7
- *CHEMICAL ABSTRACTS*, 302776-68-7
- *CHEMICAL ABSTRACTS*, 70356-09-1
- *CHEMICAL ABSTRACTS*, 103597-45-1
- *CHEMICAL ABSTRACTS*, 187393-00-6
- *CHEMICAL ABSTRACTS*, 88122-99-0
- *CHEMICAL ABSTRACTS*, 154702-15-5
- *CHEMICAL ABSTRACTS*, 155633-54-8
- *CHEMICAL ABSTRACTS*, 92761-26-7
- *CHEMICAL ABSTRACTS*, 220410-74-2
- *CHEMICAL ABSTRACTS*, 7732-18-5
- *CHEMICAL ABSTRACTS*, 64-17-5
- *CHEMICAL ABSTRACTS*, 488-86-8, 4